# EUROPEAN PATENT APPLICATION

(11) **EP 4 209 478 A1**
(43) Date of publication of application: **12.07.2023**
(21) Application number: 21864292.4
(22) Date of filing: 30.08.2021
(51) Int. Cl.: C07C 51/00, C07C 51/02, C07C 53/06, C07F 15/00, C07F 19/00, C09K 3/00, C07B 61/00, C07F 9/50, B01J 31/02, B01J 31/22, B01J 31/24

(54) **METHOD FOR PRODUCING FORMIC ACID SALT, METHOD FOR PRODUCING FORMIC ACID, CATALYST FOR PRODUCING FORMIC ACID SALT, AND RUTHENIUM COMPLEX**

(30) Priority: 03.09.2020 JP 2020148562; 12.02.2021 JP 2021021223; 12.02.2021 JP 2021021224; 12.02.2021 JP 2021021225; 10.05.2021 JP 2021079887; 17.05.2021 JP 2021083416
(71) Applicant: NITTO DENKO CORPORATION, Ibaraki-shi Osaka 567-8680 (JP)
(72) Inventor: MATSUDA Hirokazu, Ibaraki-shi, Osaka 567-8680 (JP); FUOKA Daisuke, Ibaraki-shi, Osaka 567-8680 (JP); HIRANO Makoto, Ibaraki-shi, Osaka 567-8680 (JP); PIDKO, Evgeny Alexandrovich, 3584 HT Utrecht (NL); FILONENKO, Georgy Alexandrovich, 2613 VG, Delft (NL); REBREYEND, Christophe, 1062 KS Amsterdam (NL)
(74) Representative: Hoffmann Eitle
(86) International application number: PCT/JP2021/031790
(87) International publication number: WO 2022/050234

(57) **Abstract**

The invention relates to a method for producing a formate, the method including reacting hydrogen with carbon dioxide, a hydrogen carbonate or a carbonate using a catalyst in the presence of a solvent, wherein the reaction is a two-phase system in which an organic solvent and an aqueous solvent are present in a separated state in the solvent, and the catalyst is at least one selected from a ruthenium complex represented by the formula (1) in the specification, a tautomer or stereoisomer thereof, and a salt compound of the complex, tautomer or stereoisomer.

## Description

### TECHNICAL FIELD

The present invention relates to a method for producing a formate, a method for producing formic acid, a catalyst for the production of a formate, and a ruthenium complex.

### BACKGROUND ART

In view of the problems of global warming, depletion of fossil fuel, and the like, high expectations are placed on hydrogen energy as a next-generation energy.

Formic acid is such that energy necessary for a dehydrogenation reaction is low and simple handing is possible, and therefore, formic acid is considered to be an excellent compound as a hydrogen storage material and is attracting attention.

To use formic acid as a hydrogen storage material, it is necessary to obtain a formic acid solution having high concentration in order to reduce transportation costs.

In view of the above, a method for producing formic acid from carbon dioxide (COz) and hydrogen (H₂) in the presence of a catalyst is investigated. For example, Non-Patent Literature 1 describes a method for producing formic acid by the reaction of carbon dioxide with hydrogen in the presence of a metal complex catalyst in a hydrogenation reactor.

Furthermore, the technology for efficiently extracting hydrogen from formic acid produced is important, and a metal complex catalyst for extracting hydrogen from formic acid is investigated in, for example, Patent Literature 1.

### CITATION LIST

### PATENT LITERATURE

Patent Literature 1: JP2016-539793A

### NON-PATENT LITERATURE

Non-Patent Literature 1: E. Pidko et al., ChemCatChem 2014, 6, 1526-1530

### SUMMARY OF INVENTION

### TECHNICAL PROBLEM

In the technology described in Patent Literature 1, a method for forming hydrogen from formic acid is investigated, and the formation of formic acid, easiness of separation and recovery of a catalyst, and the like are not investigated.

The technology described in Non-Patent Literature 1 is that a formate is formed from hydrogen and carbon dioxide using a metal complex catalyst in an amine solvent. Further improvement is necessary in separation and extraction of formic acid from a catalyst and a solvent, and the development of a catalyst that can form formic acid in higher yield is required.

Accordingly, the invention provides a method for producing a formate as a precursor of formic acid in high yield, in which a catalyst can be reused, a method for producing formic acid, a catalyst for the production of formic acid, and a ruthenium complex that can be used as a catalyst converting hydrogen into a formate in high yield.

### SOLUTION TO PROBLEM

As a result of intensive investigations, the present inventors have found that hydrogen can be converted into a formate in high efficiency by using a metal complex having a specific structure as a catalyst. The present inventors have further found a method for producing a formate, in which the catalyst can be recovered in high efficiency and reused, and a method for producing formic acid, and have completed the invention.

Means for solving the above problems are as follows.
[1] A method for producing a formate, the method comprising reacting hydrogen with carbon dioxide, a hydrogen carbonate or a carbonate using a catalyst in the presence of a solvent,
   wherein the reaction is a two-phase system in which an organic solvent and an aqueous solvent are present in a separated state in the solvent, and
   the catalyst is at least one selected from a ruthenium complex represented by the following formula (1), a tautomer or stereoisomer thereof, and a salt compound of the complex, tautomer or stereoisomer. 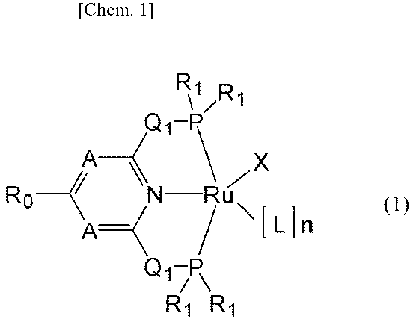
   (In the formula (1), R₀ represents a hydrogen atom or an alkyl group,
   Q₁ each independently represents CH₂, NH or O,
   R₁ each independently represents an alkyl group or an aryl group (provided that when Q₁ represents NH or O, at least one of R₁ represents an aryl group),
   A each independently represents CH, CRs or N, R₅ represents an alkyl group, an aryl group, an aralkyl group, an amino group, a hydroxy group or an alkoxy group,
   X represents a halogen atom,
   n represents 0 to 3, and
   when more than one L are present, L each independently represents a neutral or anionic ligand.)
[2] The method for producing a formate described in [1], wherein the ruthenium complex represented by the formula (1) is a ruthenium complex represented by the following formula (3). 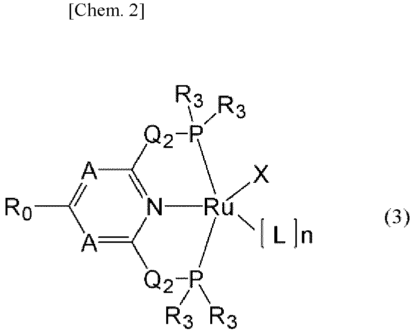
   (In the formula (3), R₀ represents a hydrogen atom or an alkyl group,
   Q₂ each independently represents NH or O,
   R₃ each independently represents an aryl group,
   A each independently represents CH, CRs or N, R₅ represents an alkyl group, an aryl group, an aralkyl group, an amino group, a hydroxy group or an alkoxy group,
   X represents a halogen atom, n represents 0 to 3, and
   when more than one L are present, L each independently represents a neutral or anionic ligand.)
[3] The method for producing a formate described in [1], wherein the R₁ represents a phenyl group.
[4] The method for producing a formate described in [2], wherein the R₃ represents a phenyl group.
[5] The method for producing a formate described in [4], wherein the A represents CH, and the Q₂ represents NH.
[6] The method for producing a formate described in any one of [1] to [5], wherein the R₀ represents a hydrogen atom or a methyl group.
[7] The method for producing a formate described in any one of [1] to [6], wherein the X represents a chlorine atom.
[8] The method for producing a formate described in any one of [1] to [7], wherein the n represents 1 to 3, and the L each independently represents a hydrogen atom, carbon monoxide or triphenylphosphine.
[9] The method for producing a formate described in any one of [1] to [8], wherein the organic solvent includes toluene or dioxane.
[10] The method for producing a formate described in any one of [1] to [9], wherein an ammonium salt is further used as a phase transfer catalyst.
[11] The method for producing a formate described in any one of [1] to [10], wherein a ligand represented by the following formula (4) is further added. 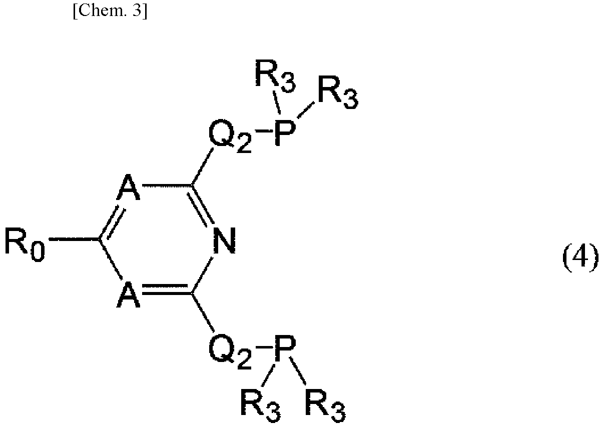
   (In the formula (4), R₀ represents a hydrogen atom or an alkyl group,
   Q₂ each independently represent NH or O,
   R₃ each independently represent an aryl group,
   A each independently represent CH, CRs or N, and R₅ represents an alkyl group, an aryl group, an aralkyl group, an amino group, a hydroxy group or an alkoxy group.)
[12] A method for producing formic acid, the method comprising:
   a step of producing a formate by the method described in any one of [1] to [11], and
   a second step of protonating at least a part of the formate to form formic acid.
[13] A catalyst for the production of a formate, for use in the production of a formate by a reaction of hydrogen with carbon dioxide, a hydrogen carbonate or a carbonate, the catalyst containing a ruthenium complex represented by the following formula (2). 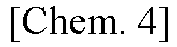 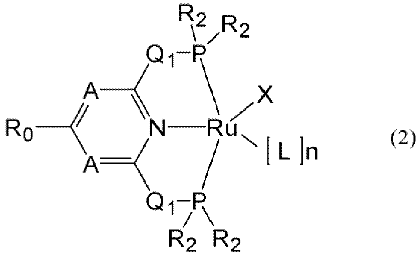
   (In the formula (2), R₀ represents a hydrogen atom or an alkyl group,
   Q₁ each independently represents CH₂, NH or O,
   R₂ each independently represents an alkyl group or an aryl group (provided that at least one of R₂ represent an aryl group),
   A each independently represents CH, CRs or N, R₅ represents an alkyl group, an aryl group, an aralkyl group, an amino group, a hydroxy group or an alkoxy group,
   X represents a halogen atom,
   n represents 0 to 3, and
   when more than one L are present, L each independently represents a neutral or anionic ligand.)
[14] A ruthenium complex represented by the following formula (3). 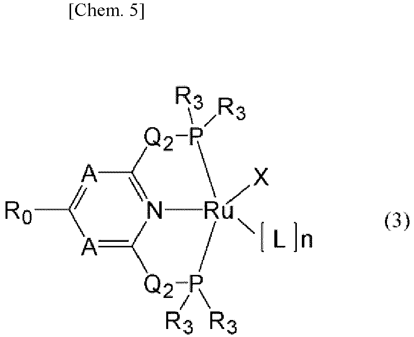
   (In the formula (3), R₀ represents a hydrogen atom or an alkyl group,
   Q₂ each independently represents NH or O,
   R₃ each independently represents an aryl group,
   A each independently represents CH, CRs or N, R₅ represents an alkyl group, an aryl group, an aralkyl group, an amino group, a hydroxy group or an alkoxy group,
   X represents a halogen atom,
   n represents 0 to 3, and
   when more than one L are present, L each independently represents a neutral or anionic ligand.)
[15] The ruthenium complex described in [14], wherein the R₃ represents a phenyl group.
[16] The ruthenium complex described in [14] or [15], wherein the A represents CH, and the Q₂ represents NH.
[17] The ruthenium complex described in any one of [14] to [16], wherein the R₀ represents a hydrogen atom or a methyl group.
[18] The ruthenium complex described in any one of [14] to [17], wherein the X represents a chlorine atom.
[19] The ruthenium complex described in any one of [14] to [18], wherein the n represents 1 to 3, and the L each independently represents a hydrogen atom, carbon monoxide or triphenylphosphine.

### ADVANTAGEOUS EFFECTS OF INVENTION

According to the invention, a ruthenium complex that can be used as a catalyst converting hydrogen into a formate with high efficiency, a catalyst for the production of a formate, and a method for producing a formate, in which a formate is produced in high yield and a catalyst can be reused, and a method for producing formic acid, can be provided.

### BRIEF DESCRIPTION OF DRAWINGS

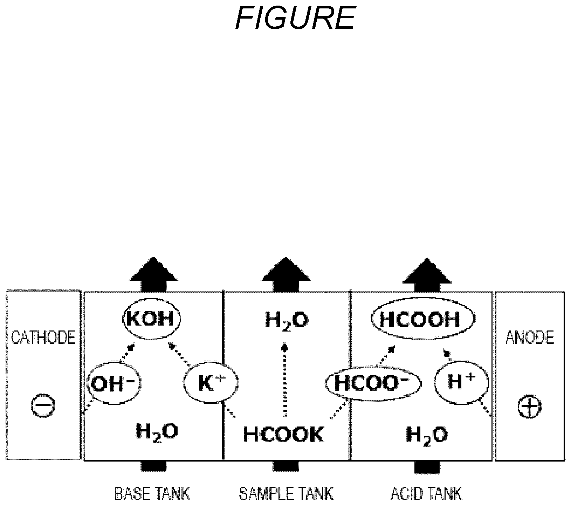

The FIGURE is a schematic diagram illustrating an example of a three-chamber type electrodialyzer.

### DESCRIPTION OF EMBODIMENTS

Embodiments of the invention are described in detail below.

A method for producing a formate according to a first embodiment of the invention is a method for producing a formate, the method including reacting hydrogen with carbon dioxide, a hydrogen carbonate or a carbonate using a catalyst in the presence of a solvent,
wherein the reaction is carried out in a two-phase system in which an organic solvent and an aqueous solvent are present in a separated state in the solvent, and
the catalyst is at least one selected from a ruthenium complex represented by the following formula (1), a tautomer or stereoisomer thereof, and a salt compound of the complex, tautomer or stereoisomer. 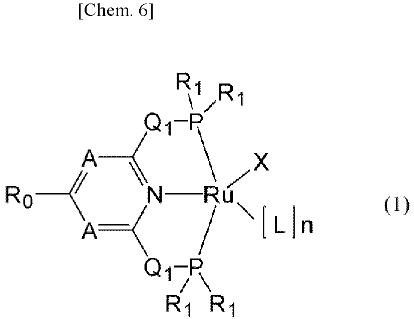
In the formula (1), R₀ represents a hydrogen atom or an alkyl group,
Q₁ each independently represents CH₂, NH or O,
R₁ each independently represents an alkyl group or an aryl group (provided that when Q₁ represents NH or O, at least one of R₁ represents an aryl group),
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

A method for producing formic acid according to a second embodiment of the invention includes a step of producing a formate by the above-described method for producing a formate, and a second step of protonating at least a part of the formate by electrodialysis to form formic acid and water.

A catalyst for the production of a formate according to a third embodiment of the invention is a catalyst for use in the production of a formate by a reaction of hydrogen with carbon dioxide, a hydrogen carbonate or a carbonate, the catalyst containing a ruthenium complex represented by the following formula (2). 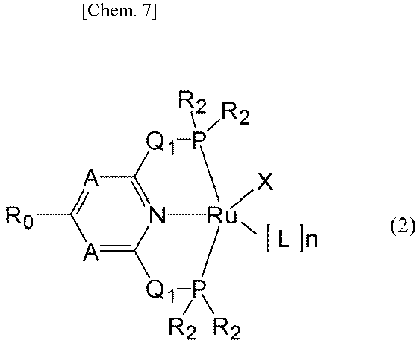
(In the formula (2), R₀ represents a hydrogen atom or an alkyl group,
Q₁ each independently represents CH₂, NH or O,
R₂ each independently represents an alkyl group or an aryl group (provided that at least one of R₂ represents an aryl group),
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

A ruthenium complex according to a fourth embodiment of the invention is represented by the following formula (3). 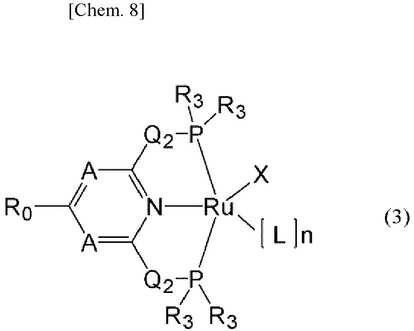
(In the formula (3), R₀ represents a hydrogen atom or an alkyl group,
Q₂ each independently represents NH or O,
R₃ each independently represents an aryl group,
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

The ruthenium complexes represented by the formulae (1) to (3) may form stereoisomers due to coordination of a ligand or conformation, but may be a mixture of these stereoisomers or may be a single pure isomer.

### [Method for producing formate and method for producing formic acid]

The method for producing a formate according to the first embodiment of the invention is a method for producing a formate, the method including reacting hydrogen with carbon dioxide, a hydrogen carbonate or a carbonate using a catalyst in the presence of a solvent,
wherein the reaction is carried out in a two-phase system in which an organic solvent and an aqueous solvent are present in a separated state in the solvent, and
the catalyst is at least one (hereinafter, sometimes simply referred to as a "ruthenium complex") selected from a ruthenium complex represented by the following formula (1), a tautomer or stereoisomer thereof, and a salt compound of the complex, tautomer or stereoisomer.

The method for producing formic acid according to the second embodiment of the invention includes a step (first step) of producing a formate by the method for producing a formate according to the first embodiment, and a second step of protonating at least a part of the formate by electrodialysis to form formic acid and water.

### <First step>

The first step is a step of reacting hydrogen with carbon dioxide, a hydrogen carbonate or a carbonate using a catalyst in the presence of a solvent and producing a formate in the reaction liquid.

In the first embodiment of the invention, the reaction of hydrogen with carbon dioxide, a hydrogen carbonate or a carbonate is carried out in a two-phase system in which an organic solvent and aqueous solvent are present in a separated state in the solvent, and is preferably conducted in a solution containing a catalyst, in which the catalyst is dissolved in an organic solvent.

The method for producing a formate according to the first embodiment of the invention can be conducted, for example, as follows. A reaction vessel equipped with a stirring device is provided, and a solvent is introduced into the reaction vessel. As necessary, a phase transfer catalyst may be further added. A catalyst is added to the reaction vessel and dissolved in a solvent to prepare a catalyst solution. Hydrogen and carbon dioxide, a hydrogen carbonate or a carbonate are introduced into the reaction vessel, and a reaction is conducted.

### (Solvent)

The solvent according to the embodiment of the invention is not particularly limited so long as it can be obtained as a two-phase system in which an organic solvent and an aqueous solvent are present in a separated state in the solvent, and preferably contains a solvent that dissolves the catalyst to form a uniform solution.

The aqueous solvent includes, for example, water, methanol, ethanol, ethylene glycol, glycerin and mixed solvents thereof. Water is preferred from the standpoint of low environmental load.

The organic solvent includes, for example, toluene, benzene, xylene, propylene carbonate, dioxane, dimethyl sulfoxide and mixed solvents thereof. It is preferable to include toluene or dioxane from the standpoint of separability from the aqueous solvent, and toluene is more preferred.

### (Catalyst)

The catalyst used in the method for producing a formate according to the first embodiment of the invention is a ruthenium complex represented by the formula (1) as described above. The ruthenium complex represented by the formula (1) is dissolved in an organic solvent and is insoluble in water. A formate formed by the reaction is easily dissolved in water. Therefore, the separation of the catalyst and the formate is easily achieved by a two-phase system reaction, the catalyst and the formate are respectively easily separated and recovered from the reaction system, and this enabled production of a formate in high yield.

According to the method of the present embodiment, a formate formed by the reaction can be separated from the catalyst by simple operation, and expensive catalyst can be reused.

The catalyst used in the embodiment of the invention is at least one selected from a ruthenium complex represented by the following formula (1), a tautomer or stereoisomer thereof, and a salt compound of the complex, tautomer or stereoisomer. 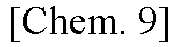 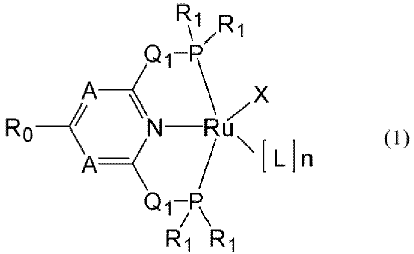
(In the formula (1), R₀ represents a hydrogen atom or an alkyl group,
Q₁ each independently represents CH₂, NH or O,
R₁ each independently represents an alkyl group or an aryl group (provided that when Q₁ represents NH or O, at least one of R₁ represents an aryl group),
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

The R₀ in the formula (1) represents a hydrogen atom or an alkyl group. The alkyl group represented by R₀ includes a straight chain, branched or cyclic substituted or unsubstituted alkyl group.

The alkyl group represented by R₀ preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. From the standpoint of easy procurement of raw materials, an alkyl group having 6 or fewer carbon atoms is preferred, and a methyl group is preferred.

The R₀ in the formula (1) is preferably a hydrogen atom or a methyl group.

R₁ in the formula (1) each independently represents an alkyl group or an aryl group, provided that when Q₁ represents NH or O, at least one of R₁ represents an aryl group.

The alkyl group represented by R₁ includes a straight chain, branched or cyclic substituted or unsubstituted alkyl group. The alkyl group represented by R₁ preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. From the standpoint of catalyst activity, an alkyl group having 12 or fewer carbon atoms is preferred, and a t-butyl group is preferred.

The aryl group represented by R₁ includes a substituted or unsubstituted aryl group having 6 to 30 carbon atoms, such as a phenyl group, a p-tolyl group, a naphthyl group, an m-chlorophenyl group or an o-hexadecanoylaminophenyl group. An aryl group having 12 or fewer carbon atoms is preferred, and a phenyl group is more preferred.

A each independently represents CH, CRs or N, and R₅ represents an alkyl group, an aryl group, an aralkyl group, an amino group, a hydroxy group or an alkoxy group.

The alkyl group represented by R₅ includes a straight chain, branched or cyclic substituted or unsubstituted alkyl group. The alkyl group represented by R₅ preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. From the standpoint of easy procurement of raw materials, an alkyl group having 12 or fewer carbon atoms is preferred, and a methyl group is preferred.

The aryl group represented by R₅ includes a substituted or unsubstituted aryl group having 6 to 30 carbon atoms, such as a phenyl group, a p-tolyl group, a naphthyl group, an m-chlorophenyl group or an o-hexadecanoylaminophenyl group. An aryl group having 12 or fewer carbon atoms is preferred, and a phenyl group is more preferred.

The aralkyl group represented by R₅ includes a substituted or unsubstituted aralkyl group having 30 or less carbon atoms, such as a trityl group, a benzyl group, a phenethyl group, a tritylmethyl group, a diphenylmethyl group or a naphthylmethyl group, and is preferably an aralkyl group having 12 or fewer carbon atoms.

The alkoxy group represented by R₅ preferably includes a substituted or unsubstituted alkoxy group having 1 to 30 carbon atoms, such as a methoxy group, an ethoxy group, an isopropoxy group, a t-butoxy group, an n-octyloxy group or a 2-methoxyethoxy group.

X represents a halogen atom and is preferably a chlorine atom.

n represents an integer of 0 to 3 and represents the number of ligands coordinating to ruthenium. From the standpoint of stability of the catalyst, n is preferably 2 or 3.

When more than one L are present, L each independently represents a neutral or anionic ligand.

The neutral ligand represented by L includes, for example, ammonia, carbon monoxide, phosphines (for example, triphenylphosphine or tris(4-methoxyphenyl)phosphine), phosphine oxides (for example, triphenyl phosphine oxide), sulfides (for example, dimethyl sulfide), sulfoxides (for example, dimethyl sulfoxide), ethers (for example, diethyl ether), nitriles (for example, p-methylbenzonitrile), and heterocyclic compounds (for example, pyridine, N,N-dimethyl-4-aminopyridine, tetrahydrothiophene or tetrahydrofuran), and is preferably triphenylphosphine.

The anionic ligand represented by L includes, for example, a hydride ion (hydrogen atom), a nitrate ion and a cyanide ion, and is preferably a hydride ion (hydrogen atom).

In the formula (1), A preferably represents CH, and Q₁ preferably represents NH.

Furthermore, n preferably represents 1 to 3, and L each independently preferably represents a hydrogen atom, carbon monoxide or triphenylphosphine.

The ruthenium complex represented by the formula (1) may be used singly alone and may be used as a mixture of two or more kinds.

The ruthenium complex represented by the formula (1) is preferably a ruthenium complex represented by the following formula (3). 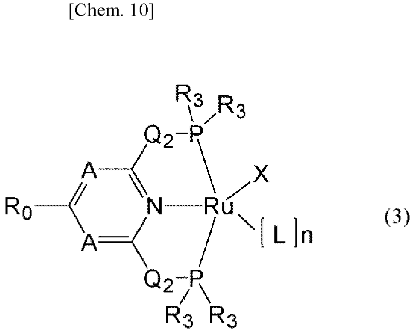
(In the formula (3), R₀ represents a hydrogen atom or an alkyl group,
Q₂ each independently represents NH or O,
R₃ each independently represents an aryl group,
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

R₀, A, R₅, X, n and L in the formula (3) are synonymous with R₀, A, R₅, X, n and L in the formula (1), respectively, and preferred ranges thereof are also the same.

The aryl groups represented by R₃ in the formula (3) are each synonymous with the aryl group represented by R₁ in the formula (1), and the preferred ranges thereof are also the same.

In the formula (3), A preferably represents CH, and Q₂ preferably represents NH.

Regarding the ruthenium complexes represented by the formulae (1), (2) and (3), ruthenium complexes produced by conventional methods can be used. As the conventional methods, for example, the methods described in Non-Patent Literature 1 and the like can be used.

The amount of the ruthenium complex used as a catalyst is not particularly limited so long as a formate can be produced. The amount of the ruthenium complex used as a catalyst is preferably 0.1 µmol or more, more preferably 0.5 µmol or more, and still more preferably 1 µmol or more, per 1 L of an organic phase (organic solvent) and an aqueous phase solvent (aqueous solvent) in order to sufficiently express catalyst function. Furthermore, from the standpoint of cost, the amount is preferably 1 mol or less, preferably 10 mmol or less, and still more preferably 1 mmol or less. When two or more kinds of ruthenium complexes are used, the total amount of those used only needs to be in the above range.

In the method for producing a formate according to the embodiment of the invention, a ligand forming the complex represented by the formula (1) is preferably present in excess in the reaction mixture. Therefore, the ligand of the complex used is preferably further added.

That is, in the method for producing a formate according to the embodiment of the invention, a ligand represented by the following formula (4) is preferably further added. 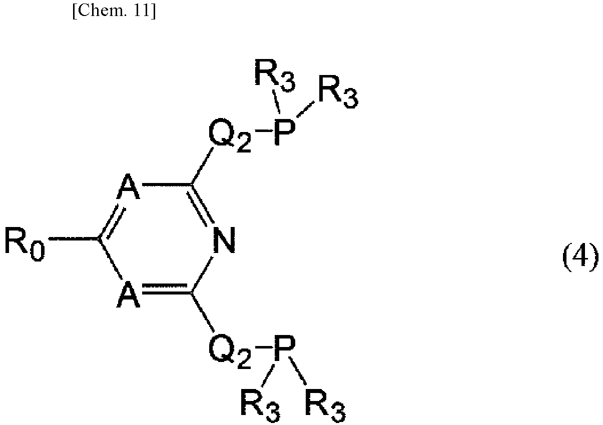
(In the formula (4), R₀ represents a hydrogen atom or an alkyl group,
Q₂ each independently represents NH or O,
R₃ each independently represents an aryl group,
A each independently represents CH, CRs or N, and R₅ represents an alkyl group, an aryl group, an aralkyl group, an amino group, a hydroxy group or an alkoxy group.)

R₀, Q₂, R₃, A and R₅ in the formula (4) are synonymous with R₀, Q₂, R₃, A, and R₅ in the formula (3), respectively, and preferred ranges thereof are also the same.

By adding the ligand for forming a complex to the reaction system in excess, even when the ligand is oxidized and deteriorated by oxygen and impurities included in the system, the deteriorated ligand and the added ligand are exchanged to restore the catalyst function, and therefore, stability of the catalyst can be improved.

Addition of the ligand represented by the above formula (4) into the reaction mixture may be carried out when the reaction mixture is prepared or may be carried out in the middle of the reaction. However, from the standpoint of process management, the addition is preferably carried out when the reaction mixture is prepared.

### (Phase transfer catalyst)

The method for producing a formate according to the first embodiment of the invention requires to conduct the reaction in a two-phase system. Therefore, a phase transfer catalyst may be used in order to smoothly perform the transfer of a substance between two phases. The phase transfer catalyst includes, for example, a quaternary ammonium salt (ammonium salt), a quaternary phosphate, a macrocyclic polyether such as a crown ether, a nitrogen-containing macrocyclic polyether such as a cryptand, a nitrogen-containing chain polyether, polyethylene glycol and an alkyl ether thereof. Above all, a quaternary ammonium salt is preferred from the standpoint that mass transfer between an aqueous solvent and an organic solvent is easy even under mild reaction conditions.

The quaternary ammonium salt includes, for example, methyltrioctylammonium chloride, benzyltrimethylammonium chloride, benzyltriethylammonium chloride, tetrabutylammonium hydroxide, tetrabutylammonium fluoride, tetrabutylammonium bromide, tetrabutylammonium iodide, trimethylphenylammonium bromide, tributylammonium tribromide, tetrahexylammonium hydrogen sulfate, decyltrimethylammonium bromide, diallyldimethylammonium chloride, dodecyltrimethylammonium bromide, dimethyldioctadecylammonium bromide, tetraethylammonium tetrafluoroborate, ethyltrimethylammonium iodide tris(2-hydroxyethyl)methylammonium hydroxide, tetramethylammonium acetate, tetramethylammonium bromide, and tetraethylammonium iodide. Methyltrioctylammonium chloride is preferred.

The amount of the phase transfer catalyst used is not particularly limited so long as a formate can be produced. The amount of the phase transfer catalyst used is preferably 0.1 mmol or more, more preferably 0.5 mmol or more and still more preferably 1 mmol or more, per 1 L of the organic phase and aqueous phase solvents for the purpose of efficiently supporting the transfer of a carbonate or a hydrogen carbonate. Furthermore, from the standpoint of cost, the amount is preferably 1 mol or less, more preferably 500 mmol or less and still more preferably 100 mmol or less. When two or more kinds of the phase transfer catalysts are used, the total amount of those needs to be in the above range.

### (Carbon dioxide and hydrogen)

As hydrogen used in the embodiment of the invention, either a hydrogen gas cylinder or liquid hydrogen can be used. As a hydrogen supply source, for example, hydrogen generated during a smelting process of iron manufacture, hydrogen generated during a soda manufacturing process, and the like can be used. Furthermore, hydrogen generated from electrolysis of water can be used.

Carbon dioxide used in the embodiment of the invention may be pure carbon dioxide gas or may be a mixed gas containing a component other than carbon dioxide. Carbon dioxide gas and other gas may be separately introduced, and a mixed gas may be formed beforehand and introduced.

The component other than carbon dioxide includes an inert gas such as nitrogen or argon, water vapor, and any optional component contained in an exhaust gas or the like.

As the carbon dioxide, a carbon dioxide gas cylinder, liquid carbon dioxide, supercritical carbon dioxide, dry ice, and the like can be used.

Hydrogen gas and carbon dioxide gas may be introduced into the reaction system each alone or may be introduced as a mixed gas.

The proportions of hydrogen and carbon dioxide used are preferably such that the proportions are equal amounts on a molar basis or hydrogen is in excess.

When a hydrogen cylinder is used as the hydrogen used in the method for producing a formate according to the embodiment of the invention, the pressure is preferably 0.1 MPa or more, more preferably 0.2 MPa or more and still more preferably 0.5 MPa or more, from the standpoint of sufficiently securing reactivity. Furthermore, the pressure is preferably 50 MPa or less, more preferably 20 MPa or less and still more preferably 10 MPa or less, from the standpoint that facilities are liable to become large.

The pressure of carbon dioxide used in the method for producing a formate according to the embodiment of the invention is preferably 0.1 MPa or more, more preferably 0.2 MPa or more and still more preferably 0.5 MPa or more, from the standpoint of sufficiently securing reactivity. Furthermore, the pressure is preferably 50 MPa or less, more preferably 20 MPa or less and still more preferably 10 MPa or less, from the standpoint that facilities are liable to become large.

Hydrogen gas and carbon dioxide gas may be introduced into a catalyst solution by bubbling (blowing). Furthermore, after introducing a gas including hydrogen gas and carbon dioxide gas, the catalyst solution, hydrogen gas and carbon dioxide gas may be stirred by stirring with a stirring device or by rotating the reaction vessel.

A method for introducing carbon dioxide, hydrogen, a catalyst, a solvent and the like that are used for the reaction into the reaction vessel is not particularly limited. All of the raw materials may be introduced at once, a part or all of the raw materials may be introduced stepwise, or a part or all of the raw materials may be introduced continuously. Furthermore, the method may be an introduction method combining those methods.

### (Hydrogen carbonate and carbonate)

The hydrogen carbonate and carbonate used in the first embodiment of the invention include a carbonate or hydrogen carbonate of an alkali metal or an alkaline earth metal.

The hydrogen carbonate includes, for example, sodium hydrogen carbonate and potassium hydrogen carbonate. Potassium hydrogen carbonate is preferred from the standpoint of high solubility in water.

The carbonate includes, for example, sodium carbonate, potassium carbonate, sodium potassium carbonate and sodium sesquicarbonate.

The hydrogen carbonate and carbonate can be formed by a reaction of carbon dioxide with a base. For example, the hydrogen carbonate or carbonate may be formed by introducing carbon dioxide into a basic solution.

The solvent of the basic solution for forming the hydrogen carbonate or carbonate is not particularly limited, and the solvent includes water, methanol, ethanol, N,N-dimethylformamide, dimethyl sulfoxide, tetrahydrofuran, benzene, toluene, mixed solvents of these, and the like. The solvent preferably includes water and is more preferably water.

The base used for the basic solution is not particularly limited so long as the base can react with carbon dioxide and form a hydrogen carbonate or carbonate. The base is preferably a hydroxide. For example, the base includes lithium hydrogen carbonate, sodium hydrogen carbonate, potassium hydrogen carbonate, cesium hydrogen carbonate, potassium hydroxide, sodium hydroxide, diazabicycloundecene, triethylamine, sodium hydroxide, or potassium hydroxide. Among those described above, the base is preferably a hydroxide, more preferably potassium hydroxide or sodium hydroxide, and still more preferably potassium hydroxide.

The content of the base in the basic solution is not particularly limited so long as a hydrogen carbonate and a carbonate can be produced. The content of the base is preferably 0.1 mol or more, more preferably 0.5 mol or more, and still more preferably 1 mol or more, per 1 L of the aqueous phase solvent, from the standpoint of securing the amount of the formate produced. Furthermore, the content is preferably 30 mol or less, more preferably 20 mol or less, and still more preferably 15 mol or less, from the standpoint of the reaction efficiency for the formate. However, when the content exceeds the solubility in the aqueous phase, the solution becomes turbid.

The ratio of the amounts of carbon dioxide and a base used for the reaction of carbon dioxide and a base is preferably 0.1 or more, more preferably 0.5 or more, and still more preferably 1.0 or more, as a molar ratio, from the standpoint of forming a carbonate from carbon dioxide. Furthermore, the ratio is preferably 8.0 or less, more preferably 5.0 or less, and still more preferably 3.0 or less, from the standpoint of the efficiency of utilization of carbon dioxide.

The ratio of the amounts of carbon dioxide and a base used may be a ratio of molar amounts of carbon dioxide and the base that are introduced into the reaction vessel, and is molar amount (mol) of CO₂ / molar amount (mol) of the base.

When the ratio of the amounts of carbon dioxide and the base used is in the above range, excessive input of carbon dioxide into the reaction vessel is suppressed, unreacted carbon dioxide can be suppressed to a minimal level, and the final efficiency of formic acid conversion is easily improved. Furthermore, from the reaction between carbon dioxide and the base in the same vessel, carbon dioxide is hydrogenated via a hydrogen carbonate or a carbonate, and a formate can be formed.

Unreacted carbon dioxide can be recovered from the reaction vessel and reused.

The method of introducing carbon dioxide and a base into a reaction vessel and the order of introduction are not particularly limited. However, carbon dioxide is preferably introduced after the base is introduced into the reaction vessel. Furthermore, the introduction of carbon dioxide and the base is such that introduction of one or both of them may be conducted continuously or intermittently.

The reaction temperature in the reaction for forming a hydrogen carbonate or a carbonate by a reaction between carbon dioxide and a base is not particularly limited. However, the reaction temperature is preferably 0°C or higher, more preferably 10°C or higher, and still more preferably 20°C or higher, in order to dissolve carbon dioxide in the aqueous phase. Furthermore, the reaction temperature is preferably 100°C or lower, more preferably 80°C or lower, and still more preferably 40°C or lower.

The reaction time in the reaction for forming a hydrogen carbonate or a carbonate by a reaction between carbon dioxide and a base is not particularly limited. However, for example, the reaction time is preferably 0.5 hours or more, more preferably 1 hour or more, and still more preferably 2 hours or more, from the standpoint of sufficiently securing the amount of the hydrogen carbonate or carbonate formed. Furthermore, the reaction time is preferably 24 hours or less, more preferably 12 hours or less, and still more preferably 6 hours or less, from the standpoint of cost.

The hydrogen carbonate and carbonate formed by the reaction of carbon dioxide and a base can be used for a reaction of hydrogen with a hydrogen carbonate or a carbonate in the method for producing a formate according to the embodiment of the invention. Furthermore, formation of a hydrogen carbonate or a carbonate by the reaction of carbon dioxide with a base in the reaction vessel may be conducted as introduction of a hydrogen carbonate or a carbonate in the reaction vessel in the method for producing a formate.

### (Reaction conditions)

The reaction conditions in the method for producing a formate according to the embodiment of the invention are not particularly limited, and the reaction conditions can be appropriately changed during the reaction process. The form of the reaction vessel used for the reaction is not particularly limited.

The reaction of hydrogen with carbon dioxide, a hydrogen carbonate or a carbonate in the method for producing a formate according to the embodiment of the invention includes a reaction of hydrogen with carbon dioxide, a reaction of hydrogen with a hydrogen carbonate, and a reaction of hydrogen with a carbonate.

In the reaction of hydrogen with carbon dioxide, a reaction of carbonation of carbon dioxide and a reaction for forming a formate by hydrogenation of a carbonate proceed simultaneously.

The method of introducing hydrogen, carbon dioxide, a hydrogen carbonate or a carbonate into a reaction vessel and the order of introduction are not particularly limited.

For example, in the reaction of hydrogen and carbon dioxide, hydrogen and carbon dioxide are preferably introduced simultaneously. Hydrogen and carbon dioxide may be introduced singly or may be introduced as a mixed gas. Furthermore, introduction of hydrogen and carbon dioxide is such that introduction of one or both of them may be conducted continuously or intermittently.

In the reaction of hydrogen with a hydrogen carbonate and the reaction of hydrogen with a carbonate, hydrogen is preferably introduced after the hydrogen carbonate or carbonate is introduced into the reaction vessel. Introduction of hydrogen and a hydrogen carbonate or a carbonate is such that introduction of one or both of them may be conducted continuously or intermittently.

The reaction temperature in the reaction of hydrogen with carbon dioxide, a hydrogen carbonate or a carbonate is not particularly limited. However, to efficiently proceed the reaction, the reaction temperature is preferably 30°C or higher, more preferably 40°C or higher and still more preferably 50°C or higher. Furthermore, from the standpoint of energy efficiency, the reaction temperature is preferably 200°C or lower, more preferably 150°C or lower and still more preferably 100°C or lower.

The reaction temperature can be adjusted by heating or cooling, and temperature increase by heating is preferred.

Furthermore, in the reaction of hydrogen with carbon dioxide, for example, temperature may be increased by heating after introducing hydrogen and carbon dioxide into a reaction vessel, or hydrogen may be introduced after introducing carbon dioxide into a reaction vessel and increasing temperature.

In the reaction of hydrogen with a hydrogen carbonate or a carbonate, for example, it is preferable that hydrogen is introduced after introducing (forming) a hydrogen carbonate or a carbonate into a reaction vessel and temperature is increased.

The reaction time in the reaction of hydrogen with carbon dioxide, a hydrogen carbonate or a carbonate is not particularly limited. However, for example, the reaction time is preferably 0.5 hours or more, more preferably 1 hour or more, and still more preferably 2 hours or more, from the standpoint of sufficiently securing the amount of a formate formed. Furthermore, the reaction time is preferably 24 hours or less, more preferably 12 hours or less, and still more preferably 6 hours or less, from the standpoint of cost.

### <Second step>

A second step is a step of protonating at least a part of the formate by electrodialysis to form formic acid and water.

In the embodiment of the invention, since the formate formed in the first step is eluted into the aqueous phase, an aqueous solution of formate is obtained by fractionating the aqueous phase.

Formic acid is preferably formed by separating the aqueous phase in the first step and treating the obtained aqueous solution of formate using an electrodialyzer by the second step. The aqueous phase to be separated is the aqueous phase after completion of the first step.

In the second step, the aqueous solution of formate obtained by the first step as described above may be used as it is, or the formate concentration may be adjusted by concentrating or diluting the solution as necessary and used.

A method for diluting an aqueous solution of a formate includes a method of diluting the solution by adding pure water.

A method for concentrating an aqueous solution of a formate includes a method of distilling off water from the aqueous solution of formate, a method of concentrating the aqueous solution of formate using a separation membrane unit equipped with a reverse osmosis membrane, and the like.

During the treatment using an electrodialyzer, it is preferable to separate the aqueous phase in the first step, adjust the concentration of the formate in the aqueous phase by dilution and then use the aqueous phase in the second step, from the standpoint of suppressing the formate loss caused by a phenomenon of concentration diffusion of a high concentration aqueous solution of a formate.

When an aqueous solution of a formate with high concentration is obtained by the first step, the formate concentration is adjusted by diluting to a concentration appropriate for electrodialysis, and then the aqueous solution of formate is supplied to the second step, TON is further increased, and formic acid can be produced in higher yield and with more excellent productivity.

The degree of preparation (preferably dilution) of the concentration of the aqueous solution of formate obtained in the first step can be appropriately selected. The concentration of a formate in the aqueous solution of formate after concentration adjustment is preferably a concentration appropriate for electrodialysis, preferably 2.5 mol/L or more, more preferably 3 mol/L or more, and still more preferably 5 mol/L or more. Furthermore, the concentration is preferably 20 mol/L or less, more preferably 15 mol/L or less, and still more preferably 10 mol/L or less, from the standpoint of suppressing the formate loss caused by a phenomenon of concentration diffusion of the high-concentration aqueous solution of formate during a treatment using an electrodialyzer.

Pure water can be used for dilution. Furthermore, the water formed in the second step may be used for dilution. It is preferable to reuse the water formed by the second step during dilution because there are advantages such as that the cost required for waste water treatment and the environmental load can be reduced.

In the method for producing formic acid according to the embodiment of the invention, the aqueous solution of formate obtained by the first step may be reused in the second step after adding acid to the solution and conducting a decarbonation treatment. That is, the aqueous phase in the first step is separated, acid is added to conduct a decarbonation treatment, and then the aqueous phase may be used in the second step.

The aqueous solution of formic acid salt obtained by the first step may include unreacted carbonate and hydrogen carbonate formed by side reactions, and when the solution containing carbonate and hydrogen carbonate is electrodialyzed, there is a risk that carbon dioxide may be generated, and the dialysis efficiency may be decreased. Therefore, by adding acid to the aqueous solution of formate obtained by the first step, conducting a decarbonation treatment and then electrodialyzing the solution, TON can be further increased, and formic acid can be produced in higher yield with more excellent productivity.

The acid used for the decarbonation treatment includes, for example, formic acid, citric acid, acetic acid, malic acid, lactic acid, succinic acid, tartaric acid, butyric acid, fumaric acid, propionic acid, hydrochloric acid, nitric acid and sulfuric acid. Formic acid is preferably used.

Regarding the amount of the acid used, the amount of the acid used is preferably 50% or more, and more preferably 80% or more, with respect to the amount of carbonic acid present in the solution, from the standpoint of suppressing the amount of carbonic acid generated during the electrodialysis treatment. Furthermore, from the standpoint of suppressing deterioration of the electrodialyzer by making the pH of the formate solution neutral during the electrodialysis treatment, the amount of the acid used is preferably 150% or less, and more preferably 120% or less, with respect to the amount of carbonic acid present in the solution.

In the embodiment of the invention, the proportion of the formate that is protonated by the second step is such that the proportion to be protonated is preferably 10% or more, the proportion to be protonated is more preferably 20% or more, and the proportion to be protonated is still more preferably 30% or more, with respect to the initial molar amount of the formate in the aqueous solution of formate, from the standpoint of increasing the purity of the aqueous solution of formic acid recovered.

The electrodialyzer includes a two-chamber type electrodialyzer that uses a bipolar membrane and an anionic exchange membrane or a cationic exchange membrane, a three-chamber type electrodialyzer that uses a bipolar membrane, an anionic exchange membrane and a cationic exchange membrane, and the like.

The FIGURE is a schematic diagram showing an example of a three-chamber type electrodialyzer. The electrodialyzer shown in the FIGURE is equipped with more than one bipolar membranes, more than one anionic exchange membranes, and more than cationic exchange membranes, respectively, and these bipolar membranes, anionic exchange membranes and cationic exchange membranes are disposed between an anode and a cathode, forming a base tank, a sample tank (salt tank) and an acid tank. As an aqueous solution of a formate is circulated and supplied to the sample tank while passing electricity, the formate is gradually converted to formic acid, formic acid is recovered from the acid tank, water is recovered from the sample tank, and hydroxide is recovered from the base tank.

A two-chamber type electrodialyzer is equipped with more than one bipolar membranes and more than one cationic exchange membranes, respectively, and these bipolar membranes and cationic exchange membranes are alternately disposed between an anode and a cathode. Each salt chamber is formed between each bipolar membrane and a cationic exchange membrane disposed on the cathode side thereof, while a base tank is formed between each bipolar membrane and a cationic exchange membrane disposed on the anode side thereof. By circulating and supplying an aqueous solution of a formate to the salt chamber while passing electricity, the formate circulated and supplied to the salt chamber is gradually converted to formic acid while forming hydroxide in the base tank.

A formic acid solution can be obtained by protonating the formate by a simple method by the second step.

A catalyst for production of a formate according to a third embodiment of the invention is described below.

### [Catalyst for production of formate]

The catalyst for the production of a formate according to the third embodiment of the invention is a catalyst for use in the production of a formate by the reaction of hydrogen with carbon dioxide, a hydrogen carbonate or a carbonate, and contains a ruthenium complex represented by the following formula (2). 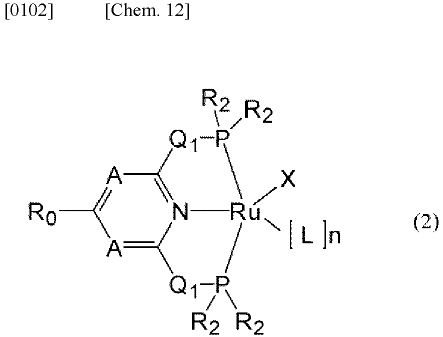
(In the formula (2), R₀ represents a hydrogen atom or an alkyl group,
Q₁ each independently represents CH₂, NH or O,
R₂ each independently represents an alkyl group or an aryl group (provided that at least one of R₂ represent an aryl group),
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

R₀, A, R₅, X, n and L in the formula (2) are synonymous with R₀, A, R₅, X, n and L in the formula (1), respectively, and preferred ranges thereof are also the same.

The alkyl group and aryl group represented by R₂ in the formula (2) are synonymous with the alkyl group and aryl group represented by R₁ in the formula (1), respectively, and preferred ranges thereof are also the same.

The ruthenium complex represented by the above formula (2) is preferably a ruthenium complex represented by the formula (3) described above.

The reaction when producing a formate using the catalyst for the production of a formate according to the third embodiment of the invention may be conducted in a single phase or may be conducted in a two-phase system.

The solvent used for the catalyst for the production of a formate according to the third embodiment of the invention is not particularly limited so long as a formate can be produced, and includes, for example, water, methanol, ethanol, ethylene glycol, glycerin, toluene, benzene, xylene, propylene carbonate, dioxane, dimethyl sulfoxide, tetrahydrofuran and mixed solvents of those.

Regarding the types and the amounts used of hydrogen, carbon dioxide, a hydrogen carbonate and a carbonate, reaction conditions, and the like when producing a formate using the catalyst for the production of a formate according to the third embodiment of the invention, those described in the method for producing a formate according to the first embodiment described above can also be appropriately adopted.

A ruthenium complex represented by the formula (3) according to a fourth embodiment of the invention is described below.

### (Ruthenium complex represented by formula (3))

The ruthenium complex represented by the formula (3) is a novel compound.

The ruthenium complex according to the fourth embodiment of the invention is represented by the following formula (3). 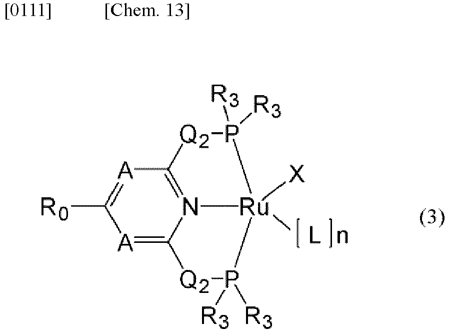
(In the formula (3), R₀ represents a hydrogen atom or an alkyl group,
Q₂ each independently represents NH or O,
R₃ each independently represents an aryl group,
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

R₀, A, R₅, X, n and L in the formula (3) are synonymous with R₀, A, R₅, X, n and L in the formula (1), respectively, and preferred ranges thereof are also the same.

The aryl group represented by R₃ in the formula (3) is synonymous with the aryl group represented by R₁ in the formula (1), and a preferred range thereof is also the same.

In the formula (3), A preferably represents CH, and Q₂ preferably represents NH.

The ruthenium complex represented by the formula (3) can be produced by, for example, adding a tridentate ligand represented by the following formula (4) and a ruthenium compound represented by the following formula (5) to a reaction system. 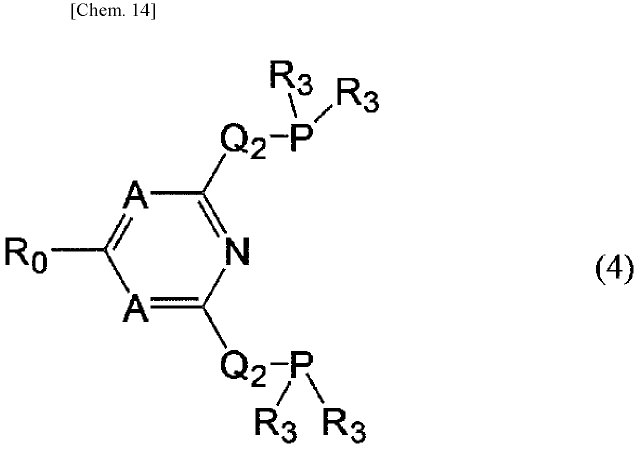
(In the general formula (4), R₀ represents a hydrogen atom or an alkyl group,
Q₂ each independently represents NH or O,
R₃ each independently represents an aryl group,
A each independently represents CH, CRs or N, and R₅ represents an alkyl group, an aryl group, an aralkyl group, an amino group, a hydroxy group or an alkoxy group.)

R₀, Q₂, R₃, A and R₅ in the formula (4) are synonymous with R₀, Q₂, R₃, A and R₅ in the formula (3), respectively, and preferred ranges thereof are also the same.

[RuHX[L]n(CO)] (5)

(In the formula (5), X represents a halogen atom, n represents 0 to 3, and when more than one L are present, L each independently represents a neutral or anionic ligand.)

X, n and L in the formula (5) are synonymous with X, n and L in the formula (3), respectively, and preferred ranges thereof are also the same.

In the production of the ruthenium complex represented by the formula (3), a solvent is preferably used. Specific examples of the solvent used include aliphatic hydrocarbons such as hexane and heptane; aromatic hydrocarbons such as benzene, toluene and xylene; halogenated hydrocarbons such as methylene chloride and chlorobenzene; ethers such as diethyl ether, tetrahydrofuran, methyl tert-butyl ether and cyclopentyl methyl ether; alcohols such as methanol, ethanol, isopropyl alcohol, n-butyl alcohol, 2-butanol and tert-butyl alcohol; polyhydric alcohols such as ethylene glycol, propylene glycol, 1,2-propanediol and glycerin; amides such as dimethylformamide and dimethylacetamide; nitriles such as acetonitrile; sulfoxides such as dimethyl sulfoxide; and water. Tetrahydrofuran is preferred.

These solvents may be used singly or may be used in appropriate combination of two or more kinds.

The production of the ruthenium complex represented by the formula (3) is desirably conducted in an inert gas or air atmosphere. The inert gas includes, for example, an argon gas and a nitrogen gas. Argon gas is preferred. Those gases and the air may be used singly or may be used as a mixed gas.

The reaction temperature is appropriately selected from a range of generally -50°C to 300°C, preferably -20°C to 250°C and more preferably 30°C to 200°C.

The reaction time varies depending on a base, a solvent, a reaction temperature and other conditions, but is appropriately selected from a range of generally 1 minute to 72 hours, preferably 1 minute to 24 hours and still more preferably 5 minutes to 12 hours.

The ruthenium complex represented by the formula (3) produced by the above production method can be subjected to a post-treatment, isolation and refinement, as necessary. Specific examples of the post-treatment method include concentration, solvent substitution, cleaning, extraction, back extraction, filtration and crystallization by addition of poor solvent. These can be conducted alone or in combination. Specific examples of the isolation and refinement methods include drying of a reaction solution, column chromatography, recrystallization and crystal cleaning with poor solvent. These can be conducted singly or in combination.

The ruthenium complex represented by the formula (3) is suitable for industrial use, and the reaction can be conducted under mild reaction conditions with high catalytic activity. For example, a formate can be produced by hydrogenation reduction of carbon dioxide or a hydrogen carbonate in the presence of a hydrogen donor.

### EXAMPLES

The invention is described in detail below by reference to Examples and Comparative Examples. However, it should be understood that the invention is not limited to those Examples.

### [Synthesis of catalyst]

### (Synthesis Example 1) Synthesis of Ru catalyst 1

Ru catalyst 1 was synthesized by the following operation.

40 mg (0.1 mmol) of a ligand A shown below was added to a THF (tetrahydrofuran) (5 ml) suspension of 95.3 mg (0.1 mmol) of [RuHCl(PPh₃)₃(CO)] in an inert atmosphere, the resulting mixture was stirred and heated at 65°C for 3 hours to conduct a reaction. Thereafter, the resulting reaction mixture was cooled to room temperature (25°C).

A yellow solution obtained was filtered, and the filtrate was evaporated to dryness under a vacuum. Yellow residual oil obtained was dissolved in THF (1 mL), hexane (10 mL) was slowly added to the resulting solution to precipitate a yellow solid, and the solid was filtered and dried under vacuum. Thus, Ru catalyst 1 (55 mg, 97%) as yellow crystals was obtained. In the Ru ruthenium catalyst 1 and ligand A shown below, tBu indicates a tertiary butyl group.

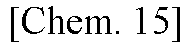

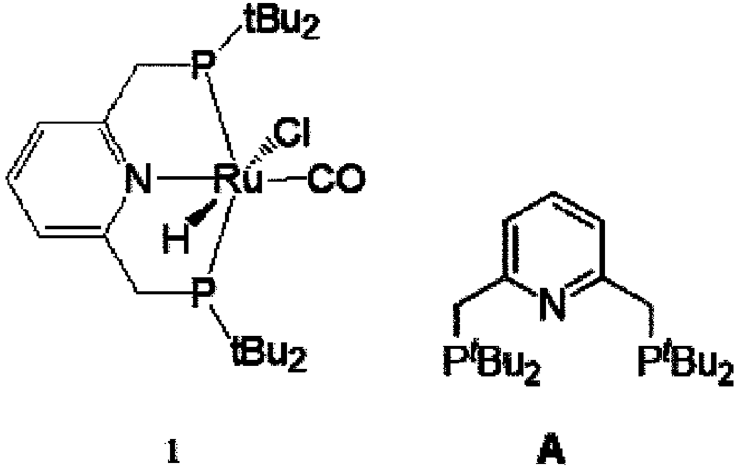

³¹P{¹H}(C₆D₆) :90.8 (s), ¹H(C₆D₆) :-14.54 (t, 1H, *J*=20.0Hz), 1.11 (t, 18H, *J*=8.0Hz), 1.51 (t, 18H, *J*=8.0Hz), 2.88 (dt, 2H, *J*=16.0Hz, *J*=4.0Hz), 3.76 (dt, 2H, *J*=16.0Hz, *J*=4.0Hz), 6.45 (d, 2H, *J*=8.0Hz), 6.79 (t, 1H, *J*=8.0Hz).¹³C{¹H}NMR(C₆D₆) :29.8 (s), 30.7 (s), 35.2 (t, *J*=9.5Hz), 37.7 (t, *J*=6.0Hz), 37.9 (t, *J*=6.5Hz), 119.5 (t, *J*=4.5Hz), 136.4 (s), 163.4 (t, *J*=5.0Hz), 209.8 (s).

### (Synthesis Example 2) Synthesis of Ru catalyst 2

Ru catalyst 2 was synthesized by the following operation.

83.9 mg (0.21 mmol) of a ligand B shown below was added to a benzene (5 mL) slurry of 200.0 mg (0.21 mmol) of [RuHCl(PPh₃)₃(CO)] in an inert atmosphere.

The resulting mixture was heated in a sealed vessel at 100°C for 8 hours, and a transparent pink solution was obtained.

The solvent was removed under a vacuum, and a pink solid was obtained.

To remove free PPh₃, the residue was cleaned with pentane (5 mL), and the cleaning liquid was passed through a silica column.

The residue was dissolved in benzene (1 mL), and the solution was passed through a column. The column was further cleaned with benzene (5 mL), and finally, the product was eluted with THF.

The solvent THF was removed under vacuum, and Ru catalyst 2 as a pink solid was obtained in a yield of 95.3%

(113 mg). In the Ru catalyst 2 and ligand B shown below, tBu indicates a tertiary butyl group.

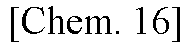

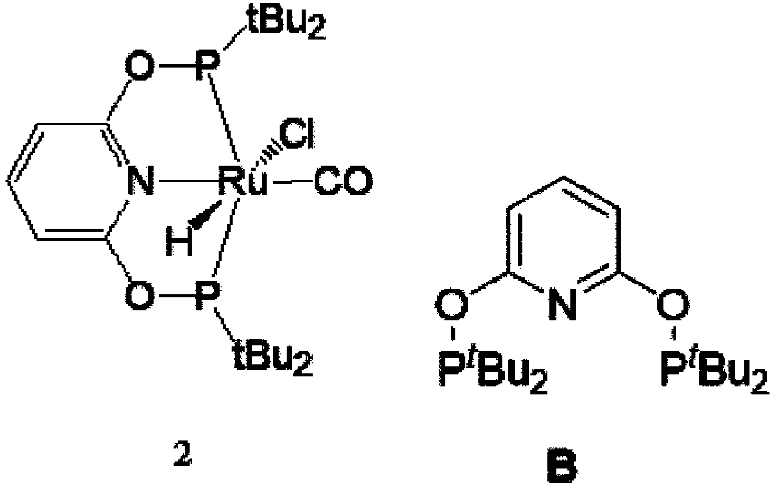

³¹P{¹H}NMR (C₆D₆): 226.65(brs).¹H NMR (C₆D₆): 6.71 (t, *J*=8.2Hz, 1H), 6.09 (d, *J*=8.2Hz, 2H), 1.72 (vt, *J*=7.6Hz), 1.20 (vt, *J*=7.3Hz), -14.07 (t, *J*=20.7Hz, 1H). ¹³C{¹H}NMR (C₆D₆): 206.84 (t, *J*=10.2Hz), 163.20 (t, *J*=3.9Hz), 142.0 (s), 102.30 (s), 43.18 (t, *J*=3.0Hz), 40.70 (t, *J*=7.6Hz), 30.50 (vt, *J*=3.8Hz), 27.85 (vt, *J*=2.8Hz).

### (Synthesis Example 3) Synthesis of Ru catalyst 3

Ru catalyst 3 was synthesized by the following operation.

A ligand C (397 mg, 1 mmol) shown below was added to a suspension of [RuHCl(PPh₃)₃(CO)] (953 mg, 1 mmol) in THF (15 ml) in an inert atmosphere. The resulting mixture was stirred and heated at 65°C for 12 hours. Thereafter, the mixture was cooled to room temperature.

A pale yellow solid precipitated was filtered, cleaned with ether (3 mL, three times) and dried under vacuum. Thus, Ru catalyst 3 was obtained (519 mg, 92%).

In the Ru catalyst 3 and ligand C shown below, tBu indicates a tertiary butyl group.

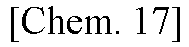

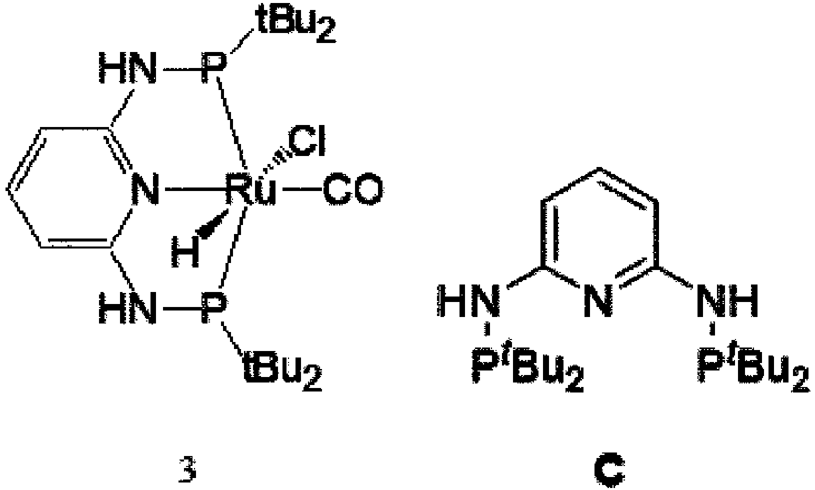

³¹P{¹H}NMR (CDCl₃): 135.6 (s).¹HNMR (CDCl3):δ-26.11 (t, *J*=16.0Hz, 1H), 1.32 (t, *J*=7.28Hz, 18H), 1.41 (t, *J*=7.68Hz, 18H), 6.87 (t, *J*=8.04Hz, 1H), 7.14 (d, *J*=8.04Hz, 2H), 9.51 (br, 2H).¹³C{¹H}NMR (125MHz, CD₃OD): 28.62 (t, *J*=2.7Hz), 28.87 (t, *J*=3.4Hz), 39.34 (t, *J*=10.8Hz), 41.10 (t, *J*=9.1Hz), 99.75 (t, *J*=3.5Hz), 143.05 (s), 164.32 (t, *J*=7.3Hz), 207.41 (t, *J*=10.7Hz).

### (Synthesis Example 4) Synthesis of Ru catalyst 5

Ru catalyst 5 was synthesized by the following operation.

A mixture of 0.51 g (0.54 mmol) of [RuHCl(PPh₃)₃(CO)] and 0.30 g (0.63 mmol) of a ligand E shown below in benzene (25 mL) was refluxed one night in an inert atmosphere, and a transparent yellow solution was obtained. The solution obtained was naturally cooled to room temperature.

The solvent was completely removed under a vacuum, and 30 mL of diethyl ether was added to obtain a yellow solid.

The yellow solid was recrystallized using dichloromethane/diethyl ether, and a pale yellow solid was obtained.

The solid was collected on a filter and dried one night under a vacuum. Thus, Ru catalyst 5 was obtained at a yield of 0.29 g, 85%.

In the Ru catalyst 5 and ligand E shown below, Ph indicates a phenyl group.

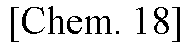

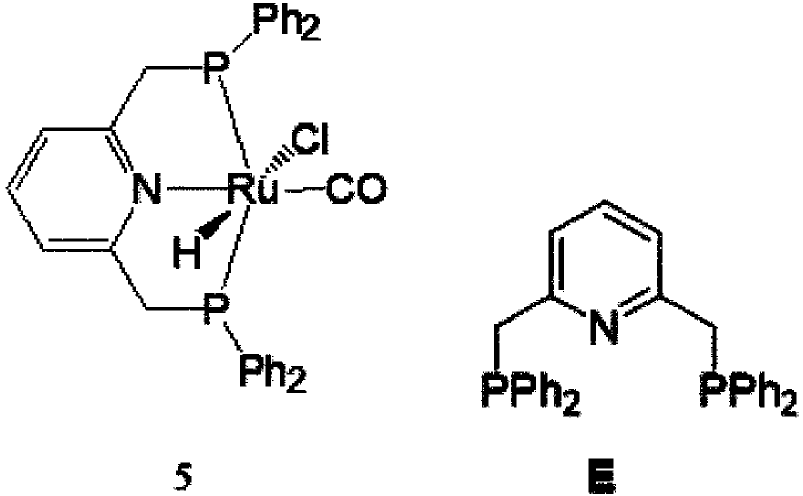

¹HNMR (300MHz, CD₂Cl₂): δ-13.65 (t, *J*=19.9Hz, 1H), 4.13 (dt, *J*=16.6Hz, *J*=4.8Hz, 2H), 4.64 (dt, *J*=16.6Hz, *J*=4.SHz, 2H), 6.82-7.85 (m, 23H).³¹P{¹H}NMR (121.51MHz, CD₂Cl₂): δ50.4 (s)

### (Synthesis Example 5) Synthesis of Ru catalyst 7

Ru catalyst 7 was synthesized by the following operation.

142.6 mg of a ligand G and 284.6 mg of [RuHCl(PPh₃)₃(CO)] were mixed with 5 mL of benzene in an inert atmosphere, and the resulting suspension was refluxed one night. A yellow precipitate formed was collected on a filter and cleaned with 5 mL of ether 4 times.

The precipitate was dried in a vacuum, and 154.0 mg of Ru catalyst 7 was obtained.

In the Ru catalyst 7 and ligand G shown below, Ph indicates a phenyl group.

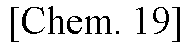

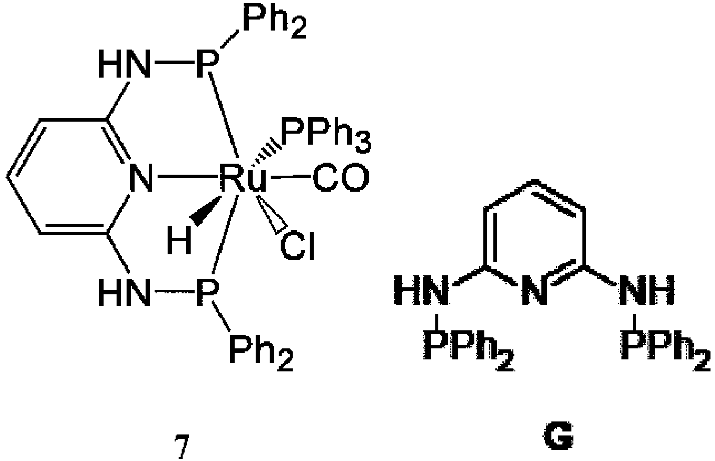

³¹P{¹H}NMR (CDC₃): 95.58 (br, s), 29.71 (s).¹H NMR (400MHz, CD₂Cl₂) δ9.92 (s, 2H), 8.11 (q, *J*=6.6Hz, 4H), 7.38-7.24 (m, 4H), 7.20(t, J=7.5Hz, 3H), 7.16-7.04 (m, 4H), 7.04-6.92 (m, 14H), 6.87 (td, *J*=7.6, 2.1Hz, 6H), 6.51 (d, *J*=8.0Hz, 1H), 6.61 (d, *J*=8.0Hz, 2H), -7.22 (dt, *J*=89.2, 23.1Hz, 1H).

### (Synthesis Example 6) Synthesis of Ru catalyst 8

Ru catalyst 8 was synthesized by the following operation.

161.0 mg of a ligand H and 242.4 mg of [RuHCl(PPh₃)₃(CO)] were mixed with 6 mL of THF in an inert atmosphere, and the resulting mixture was stirred at 64°C one night. After cooling to room temperature, the solvent was removed in a vacuum, and the residue was cleaned with 3 mL of diethyl ether two times. A pale yellow powder obtained was further dried in a vacuum, and 110.2 mg of Ru catalyst 8 was obtained.

In the Ru catalyst 8 and ligand H shown below, Ph indicates a phenyl group.

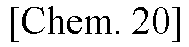

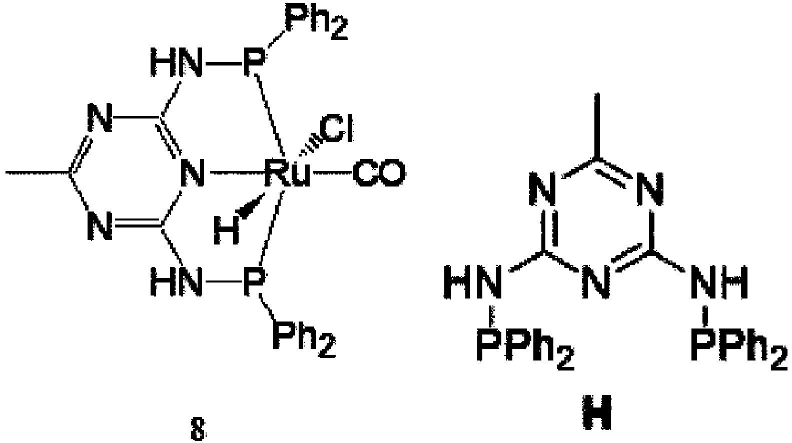

³¹PNMR (162MHz, THF-d8) δ90.19 (s).¹HNMR (400MHz, THF-d8) δ9.26 (s, 2H), 7.94 (br, 4H), 7.74 (t, J=4.6Hz, 4H), 7.43-7.32 (br12H), 2.26 (d, J=2.2Hz, 3H), -13.63 (t, J 21.6Hz, 1H).

### [Example 1]

2 mL of N,N-dimethylformamide (DMF) was put in a glass vial equipped with a rare earth metal stirring rod in a glovebox in an argon atmosphere. Thereafter, 2.23 mmol of azabicycloundecene (DBU) (333 µL) was added. Next, 10 mg of a 6.6 mg/mL stock solution of the catalyst 7 (solution obtained by dissolving 6.6 mg of the catalyst 7 in 1 mL of DMF) was added. Finally, an autoclave was sealed and taken out of the glovebox.

The autoclave was connected to an H₂/CO₂ (1:1 mixture) supply line and firstly purged, and a slight amount of oxygen and other impurities were removed. Thereafter, the autoclave was pressurized to about 5 bars (1 bar = 0.1 MPa) at room temperature and heated to a target temperature (90°C) while being stirred. When the temperature reached the target temperature, the autoclave was further pressurized to 40 bars with a H₂/CO₂ mixture (1:1).

After stirring the reaction mixture for 1.5 hours, the reaction mixture was cooled with an ice bath. After cooling, the pressure was carefully released. 300 µL of dimethyl sulfoxide (DMSO) was added as an internal standard, 100 µL of a sample was collected and dissolved in 0.5 mL of D₂O, and the amount of potassium formate formed was quantitatively determined by ¹H NMR analysis.

### [Example 2]

Potassium bicarbonate (10 mmol, 1.0 g) was weighed out and put in a glass vial equipped with a rare earth metal stirring rod in a glovebox in an argon atmosphere. Thereafter, solid methyltrioctylammonium chloride (54 µmol, 22 mg) was added. Next, 2 mL of water and 0.12 µmol (108 µg, corresponding to 10 µL as a 12.6 mg/mL solution of DMF) of the catalyst 7 were added. Finally, the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released.

100 µL of DMSO was added as an internal standard, 100 µL of an aqueous layer was collected and dissolved in 0.5 mL of D₂O, and potassium formate was quantitatively determined by ¹H NMR analysis.

### [Example 3]

Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 6.6 mg/mL stock solution of the catalyst 1 were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released. An upper layer containing the catalyst and trimethylammonium chloride was removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitatively determined by ¹H NMR analysis.

### [Example 4]

Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 6.6 mg/mL stock solution of the catalyst 5 were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitatively determined by ¹H NMR analysis.

### [Example 5]

Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 12.6 mg/mL stock solution of the catalyst 7 were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 4.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

### [Example 6]

Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 6.3 mg/mL stock solution of the catalyst 8 were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 12 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitatively determined by ¹H NMR analysis.

### [Example 7]

Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of dioxane and 10 µL of a 12.6 mg/mL stock solution of the catalyst 7 were added. Finally, 1 mL of water was added, the vial was placed in an autoclave, and the autoclave was sealed and taken out of the glovebox.

The autoclave was connected to an Hz supply line and firstly purged, and a slight amount of oxygen and other impurities were removed. Thereafter, the autoclave was pressurized to about 5 bars at room temperature and heated to a target temperature (generally 90°C) while being stirred. When the temperature reached the target temperature, the autoclave was further pressurized to 40 bars with H₂.

After stirring the reaction mixture for 4.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitatively determined by ¹H NMR analysis.

### [Example 8]

In this Example, the catalyst 1 was exposed to air in a solid state for 20 hours before use. Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 6.3 mg/mL stock solution of the catalyst 1 exposed to air were added. Finally, 1 mL of water was added, the vial was placed in an autoclave, and the autoclave was sealed and taken out of the glovebox.

The autoclave was connected to an Hz supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitatively determined by ¹H NMR analysis.

### [Example 9]

In this Example, the catalyst 5 was exposed to air in a solid state for 20 hours before use. Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 7.0 µL of a 9.0 mg/mL stock solution of the catalyst 5 exposed to air (solution obtained by dissolving 9.0 mg of the catalyst 5 in 1 mL of DMF) were added. Finally, 1 mL of water was added, the vial was placed in an autoclave, and the autoclave was sealed and taken out of the glovebox.

The autoclave was connected to an Hz supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

### [Example 10]

In this Example, the catalyst 7 was exposed to air in a solid state for 20 hours before use. Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10.5 µL of a 10.6 mg/mL stock solution of the catalyst 7 exposed to air were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to 90°C while being stirred. When the temperature reached the target temperature, the autoclave was further pressurized to 40 bars with H₂.

After stirring the reaction mixture for 4.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

### [Example 11]

In this Example, the catalyst 8 was exposed to air in a solid state for 20 hours before use. Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10.5 µL of a 5.8 mg/mL stock solution of the catalyst 8 exposed to air were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to a target temperature (generally 90°C) while being stirred. When the temperature reached the target temperature, the autoclave was further pressurized to 40 bars with H₂.

After stirring the reaction mixture for 12 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitatively determined by ¹H NMR analysis.

### [Comparative Example 1]

In this Example, the catalyst 2 was exposed to air in a solid state for 20 hours before use. Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 6.7 mg/mL stock solution of the catalyst 2 exposed to air were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to a target temperature (generally 90°C) while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as internal standard, 100 µL of an aqueous layer was collected and dissolved in 0.5 mL of D₂O, and potassium formate was quantitatively determined by ¹H NMR analysis.

### [Comparative Example 2]

In this Example, the catalyst 3 was exposed to air in a solid state for 20 hours before use. Potassium bicarbonate (5 mmol, 0.5 g) was weighed out and put in a glass vial equipped with a rare earth metal stirring rod in a glovebox in an argon atmosphere. Thereafter, solid methyltrioctylammonium chloride (54 µmol, 22 mg) was added. Next, 1 mL of toluene and 10 µL of a 6.6 mg/mL stock solution of the catalyst 3 exposed to air were added. Finally, 1 mL of water was added, the vial was placed in an autoclave, and the autoclave was sealed and taken out of the glovebox.

The autoclave was connected to an H₂ supply line and firstly purged, and a slight amount of oxygen and other impurities were removed. Thereafter, the autoclave was pressurized to about 5 bars at room temperature and heated to a target temperature (generally 90°C) while being stirred. When the temperature reached the target temperature, the autoclave was further pressurized to 40 bars with H₂.

After stirring the reaction mixture for 2.5 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as internal standard, 100 µL of an aqueous layer was collected and dissolved in 0.5 mL of D₂O, and potassium formate was quantitatively determined by ¹H NMR analysis.

### The above Examples and Comparative Examples are shown in Table 1.

TON (Turnover Number) in the table shows the amount (molar amount) of formic acid or formate formed to the amount (molar amount) of the catalyst used.

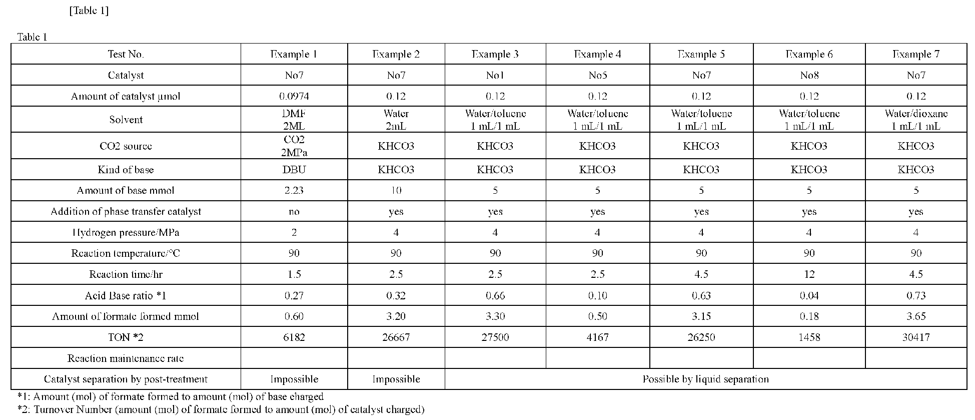

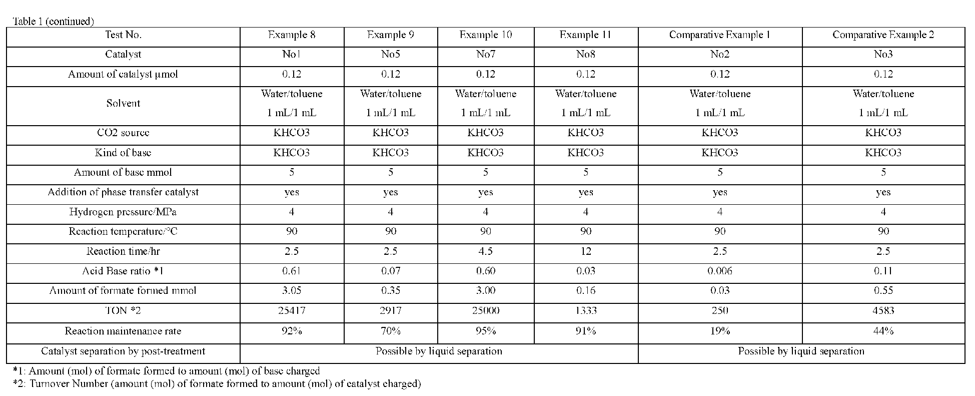

Examples 3 to 11 in which a formate was produced using the production method according to the first embodiment show high TON (Turnover Number) and are excellent in the production efficiency for a formate. It could be confirmed that Examples 1 and 2 in which a formate was produced using the ruthenium complex according to the fourth embodiment show high TON.

### [Example 12]

In this Example, the catalyst 7 was exposed to air in a solid state for 20 hours before use. Potassium hydroxide (50 mmol, 2.8 g) was weighed out and put in a glass vial equipped with a rare earth metal stirring rod in a glovebox in an argon atmosphere, and 4.2 ml of distilled water was added to obtain a 10 mol/L aqueous solution of potassium hydroxide. Next, 10.5 µL of a 10.6 mg/mL stock solution of the catalyst 7 exposed to air and solid methyltrioctylammonium chloride (54 µmol, 22 mg) were added to 5 mL of toluene. Finally, 5 mL of toluene to which the catalyst 7 and methyltrioctylammonium chloride had been added was added to 5 mL of the 10 mol/L aqueous solution of potassium hydroxide, the vial was placed in an autoclave having an internal volume of 300 mL, and the autoclave was sealed and taken out of the glovebox.

The autoclave was connected to a COz supply line and firstly purged, and a slight amount of oxygen and other impurities were removed. Thereafter, the autoclave was pressurized to about 0.4 MPa at room temperature. After the solution was stirred for 1 hour, CO₂ was carefully released.

The autoclave was connected to an H₂ supply line and firstly purged, and a slight amount of COz and other impurities were removed. Thereafter, temperature was increased to 90°C while stirring the solution, and after the temperature increase, the autoclave was pressurized to about 0.5 MPa with H₂.

After stirring the reaction mixture for 18 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

### [Example 13]

In this Example, the catalyst 7 was exposed to air in a solid state for 20 hours before use. Potassium hydroxide (50 mmol, 2.8 g) was weighed out and put in a glass vial equipped with a rare earth metal stirring rod in a glovebox in an argon atmosphere, and 4.2 ml of distilled water was added to obtain a 10 mol/L aqueous solution of potassium hydroxide. Next, 10.5 µL of a 10.6 mg/mL stock solution of the catalyst 7 exposed to air and solid methyltrioctylammonium chloride (54 µmol, 22 mg) were added to 5 mL of toluene. Finally, 5 mL of toluene to which the catalyst 7 and methyltrioctylammonium chloride had been added was added to 5 mL of the 10 mol/L aqueous solution of potassium hydroxide, the vial was placed in an autoclave having an internal volume of 300 mL, and the autoclave was sealed and taken out of the glovebox.

The autoclave was connected to a COz supply line and firstly purged, and a slight amount of oxygen and other impurities were removed. Thereafter, the autoclave was pressurized to about 0.1 MPa at room temperature. After the solution was stirred for 1 hour, CO₂ was carefully released.

The autoclave was connected to an H₂ supply line and firstly purged, and a slight amount of CO₂ and other impurities were removed. Thereafter, temperature was increased to 90°C while stirring the solution, and after the temperature increase, the autoclave was pressurized to about 0.4 MPa with H₂.

After stirring the reaction mixture for 18 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

### [Example 14]

In this Example, the catalyst 7 was exposed to air in a solid state for 20 hours before use. Potassium hydroxide (50 mmol, 2.8 g) was weighed out and put in a glass vial equipped with a rare earth metal stirring rod in a glovebox in an argon atmosphere, and 4.2 ml of distilled water was added to obtain a 10 mol/L aqueous solution of potassium hydroxide. Next, 10.5 µL of a 10.6 mg/mL stock solution of the catalyst 7 exposed to air and solid methyltrioctylammonium chloride (54 µmol, 22 mg) were added to 5 mL of toluene. Finally, 5 mL of toluene to which the catalyst 7 and methyltrioctylammonium chloride had been added was added to 5 mL of the 10 mol/L aqueous solution of potassium hydroxide, the vial was placed in an autoclave having an internal volume of 300 mL, and the autoclave was sealed and taken out of the glovebox.

The autoclave was connected to a COz supply line and firstly purged, and a slight amount of oxygen and other impurities were removed. Thereafter, the autoclave was pressurized to about 0.1 MPa at room temperature. Next, the autoclave was connected to an H₂ supply line and pressurized to about 0.4 MPa. Finally, the temperature of the solution was increased to 90°C.

After stirring the reaction mixture for 18 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

### [Example 15]

In this Example, the catalyst 7 was exposed to air in a solid state for 20 hours before use. Potassium hydroxide (50 mmol, 2.8 g) was weighed out and put in a glass vial equipped with a rare earth metal stirring rod in a glovebox in an argon atmosphere, and 4.2 ml of distilled water was added to obtain a 10 mol/L aqueous solution of potassium hydroxide. Next, 10.5 µL of a 10.6 mg/mL stock solution of the catalyst 7 exposed to air and solid methyltrioctylammonium chloride (54 µmol, 22 mg) were added to 5 mL of toluene. Finally, 5 mL of toluene to which the catalyst 7 and methyltrioctylammonium chloride had been added was added to 5 mL of the 10 mol/L aqueous solution of potassium hydroxide, the vial was placed in an autoclave having an internal volume of 300 mL, and the autoclave was sealed and taken out of the glovebox.

The autoclave was connected to a H₂ supply line and firstly purged, and a slight amount of oxygen and other impurities were removed. Thereafter, the autoclave was pressurized to about 0.1 MPa at room temperature. Next, the autoclave was connected to an COz supply line and pressurized to about 0.4 MPa. Finally, the temperature of the solution was increased to 90°C.

After stirring the reaction mixture for 18 hours, the reaction mixture was cooled with an ice bath. After cooling, the pressure was carefully released. An upper layer containing the catalyst and trimethylammonium chloride could be removed, and a lower layer containing potassium formate and unreacted potassium bicarbonate remained. 100 µL of DMSO was added as an internal standard, 100 µL of an aqueous layer was collected and dissolved in 0.5 mL of D₂O, and potassium formate was quantitively determined by ¹H NMR analysis.

The above Examples 12 to 15 are described in Table 2.

CO₂/amount of base (mol/mol) in Table 2 indicates the ratio of the amount of CO₂ (molar amount) filled in the autoclave having an internal volume of 300 mL and the amount of KOH (molar amount) used. The molar amount of the amount of CO₂ filled was calculated from the volume obtained by subtracting the volume of the input basic aqueous solution from the internal volume of the autoclave, the filling pressure of CO₂, and the environmental temperature at the time of CO₂ filling, using a gas state equation.

### [Table 2]

**Table 2**

| Test No. | Example 12 | Example 13 | Example 14 | Example 15 |
|---|---|---|---|---|
| Catalyst | No.7 | No.7 | No.7 | No.7 |
| Amount of catalyst µmol | 0.77 | 0.77 | 0.77 | 0.77 |
| Solvent | Aqueous solution/toluene 5 ml/5 ml | Aqueous solution/toluene 5 ml/5 ml | Aqueous solution/toluene 5 ml/5 ml | Aqueous solution/toluene 5 ml/5 ml |
| CO2 source | CO2 0.4 MPa | CO2 0.1 MPa | CO2 0.1 MPa | CO2 0.1 MPa |
| Kind of base | KOH | KOH | KOH | KOH |
| Amount of base mmol | 50 | 50 | 50 | 50 |
| Phase transfer catalyst | yes | yes | yes | yes |
| Hydrogen pressure/Mpa | 0.5 | 0.4 | 0.4 | 0.4 |
| Reaction temperature/°C | 90 | 90 | 90 | 90 |
| Reaction time/hr | 18 | 18 | 18 | 18 |
| Acid Base ratio *1 | 0.646 | 0.598 | 0.638 | 0.64 |
| Amount of formate formed mmol | 32.3 | 29.9 | 31.9 | 32 |
| TON *2 | 41,948 | 38,831 | 41,429 | 41,558 |
| CO2/amount of base mol/mol *3 | 9.52 | 2.4 | 2.4 | 2.4 |
| Order of charging reaction gas and temperature increase | CO2 filling (carbonation) → CO2 leak → H2 filling → temperature increase → (hydrogenation) | CO2 filling (carbonation) → CO2 leak → H2 filling → temperature increase → (hydrogenation) | CO2 filling → H2 filling → temperature increase → (carbonation + hydrogenation) | H2 filling → CO2 filling → temperature increase → (carbonation + hydrogenation) |

| | | | | |
|---|---|---|---|---|
| *1: Amount (mol) of formate formed to amount (mol) of base charged *2: Turnover Number (amount (mol) of formate formed to amount (mol) of catalyst charged) *3: Ratio of amount (mol) of CO2 charged and amount (mol) of base charged | | | | |

Example 12 in which formic acid was produced using the production method according to the first embodiment show high TON (Turnover Number (amount (mol) of formic acid formed to the amount (mol) of catalyst charged)), and it could be confirmed that formic acid is formed by a reaction between hydrogen and carbon dioxide using potassium hydroxide as a base. Furthermore, Example 13 show, even when the filling pressure of CO₂ was 1 MPa, high TON (Turnover Number (amount (mol) of formic acid formed to the amount (mol) of catalyst charged)) depending on the ratio of CO₂/amount of base, and it could be confirmed that formic acid is formed from hydrogen and carbon dioxide.

Example 14 and Example 15, in which formic acid was produced using the production method according to the first embodiment, show high TON (Turnover Number (amount (mol) of formic acid formed to the amount (mol) of catalyst charged)), and it could be confirmed that formic acid is formed by a reaction between hydrogen and carbon dioxide using potassium hydroxide as a base. The timing for increasing the temperature of the solution is not particularly limited. However, it is preferable to heat the solution after introducing hydrogen and carbon dioxide into the reaction vessel.

### [Example 16]

5 mL of water was weighed out and put in a glass vial equipped with a stirring rod in a glovebox under an inert gas, 5 mmol of calcium carbonate was added, and thereafter a solution obtained by mixing 0.6 µmol of the Ru catalyst 7 and 270 µmol of methyltrioctylammonium chloride with 5 mL of toluene was added. Thereafter, the vial was placed in an autoclave, and the autoclave was sealed and taken out of the glovebox.

The autoclave was heated to 90°C while being stirred. When the temperature reached the target temperature, a gas including 50 vol% of hydrogen and 50 vol% of carbon dioxide was put into the autoclave to pressurize the autoclave to 4 MPa. The reaction mixture was stirred for 4.5 hours, thereafter the reaction mixture was cooled with an ice bath, and pressure was carefully released. An organic phase (solution containing a homogeneous catalyst) of the solution after reaction was separated, and unreacted calcium carbonate precipitated in the aqueous phase was removed to obtain an aqueous solution containing calcium formate. Thereafter, 100 µL of the aqueous solution containing calcium formate was collected and dissolved in 0.5 mL of D₂O, 100 µL of DMSO was added as an internal standard, and calcium formate was quantitively determined by ¹H NMR analysis.

### [Example 17]

5 mL of water was weighed out and put in a glass vial equipped with a stirring rod in a glovebox under an inert gas, 5 mmol of calcium carbonate was added, and thereafter a solution obtained by mixing 0.6 µmol of the Ru catalyst 7 and 270 µmol of methyltrioctylammonium chloride with 5 mL of toluene was added. Thereafter, the vial was placed in an autoclave, and the autoclave was sealed and taken out of the glovebox.

The autoclave was heated to 90°C while being stirred. When the temperature reached the target temperature, a gas including 50 vol% of hydrogen and 50 vol% of carbon dioxide was put into the autoclave to pressurize the autoclave to 4 MPa. The reaction mixture was stirred for 18 hours, thereafter the reaction mixture was cooled with an ice bath, and pressure was carefully released. An organic phase (solution containing a homogeneous catalyst) of the solution after reaction was separated, and unreacted calcium carbonate precipitated in the aqueous phase was removed to obtain an aqueous solution containing calcium formate. Thereafter, 100 µL of the aqueous solution containing calcium formate was collected and dissolved in 0.5 mL of D₂O, 100 µL of DMSO was added as an internal standard, and calcium formate was quantitively determined by ¹H NMR analysis.

The above Examples 16 to 17 are described in Table 3.

### [Table 3]

**Table 3**

| Test No. | Example 16 | Example 17 |
|---|---|---|
| Catalyst | No.7 | No.7 |
| Amount of catalyst µmol | 0.6 | 0.6 |
| Solvent | Water/toluene 5 ml/5 ml | Water/toluene 5 ml/5 ml |
| CO2 source | CaCO3 | CaCO3 |
| | CO2 2 MPa | CO2 2 MPa |
| Kind of base | CaCO3 | CaCO3 |
| Amount of base mmol | 5 | 5 |
| Phase transfer catalyst | yes | yes |
| Hydrogen pressure/MPa | 2 | 2 |
| Reaction temperature/°C | 90 | 90 |
| Reaction time/hr | 4.5 | 18 |
| Acid Base ratio *1 | 0.07 | 0.09 |
| Amount of formate formed mmol | 0.35 | 0.45 |
| TON *2 | 583 | 750 |

| | | |
|---|---|---|
| *1: Amount (mol) of formate formed to amount (mol) of base charged *2: Turnover Number (amount (mol) of formate formed to amount (mol) of catalyst charged) | | |

Example 16 and Example 17, in which formic acid was produced using the production method according to the first embodiment, show high TON (Turnover Number (amount (mol) of formic acid formed to the amount (mol) of catalyst charged)), and it could be confirmed that formic acid can be efficiently formed by a reaction of hydrogen and an alkaline earth metal salt with carbon dioxide or a carbonate using calcium carbonate as a base.

### [Examples 18 to 21]

1 mL of water was weighed out and put in a glass vial equipped with a stirring rod in a glovebox under an inert gas, potassium hydrogen carbonate was added in an amount in the range of 2.5 to 14 mmol, and thereafter a solution obtained by mixing 0.12 µmol of the Ru catalyst 1 and 54 µmol of methyltrioctylammonium chloride with 1 mL of toluene was added. Thereafter, the vial was placed in an autoclave, and the autoclave was sealed and taken out of the glovebox.

The autoclave was heated to 90°C while being stirred. When the temperature reached the target temperature, the autoclave was pressurized to 4 MPa with hydrogen. The reaction mixture was stirred for 2.5 hours, thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 µL of the aqueous solution of the lower layer was collected and dissolved in 500 µL of deuterated water, 300 µL of dimethyl sulfoxide was added as an internal standard, and then potassium formate was quantitively determined by ¹H NMR analysis.

### [Examples 22 to 24]

1 mL of water was weighed out and put in a glass vial equipped with a stirring rod in a glovebox under an inert gas, sodium hydrogen carbonate was added in an amount in the range of 5 to 10 mmol, and thereafter a solution obtained by mixing 0.12 µmol of the Ru catalyst 1 and 54 µmol of methyltrioctylammonium chloride with 1 mL of toluene was added. Thereafter, the vial was placed in an autoclave, and the autoclave was sealed and taken out of the glovebox.

The autoclave was heated to 90°C while being stirred. When the temperature reached the target temperature, the autoclave was pressurized to 4 MPa with hydrogen. The reaction mixture was stirred for 2.5 hours, thereafter the reaction mixture was cooled with an ice bath, and pressure was carefully released. An upper layer of the solution after the reaction was removed, and an aqueous solution of a lower layer containing sodium formate and unreacted potassium hydrogen carbonate remained. 100 µL of the aqueous solution of the lower layer was collected and dissolved in 500 µL of deuterated water, 300 µL of dimethyl sulfoxide was added as an internal standard, and then sodium formate was quantitively determined by ¹H NMR analysis.

The above Examples 18 to 24 are described in Table 4.

### [Table 4]

**Table 4**

| Test No. | Example 18 | Example 19 | Example 20 | Example 21 | Example 22 | Example 23 | Example 24 |
|---|---|---|---|---|---|---|---|
| Catalyst | Catalyst No. 1 | Catalyst No. 1 | Catalyst No. 1 | Catalyst No. 1 | Catalyst No. 1 | Catalyst No. 1 | Catalyst No. 1 |
| Amount of catalyst µmol | 0.12 | 0.12 | 0.12 | 0.12 | 0.12 | 0.12 | 0.12 |
| Solvent | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL |
| CO₂ source | KHCO₃ | KHCO₃ | KHCO₃ | KHCO₃ | NaHCO₃ | NaHCO₃ | NaHCO₃ |
| Kind of base | KHCO₃ | KHCO₃ | KHCO₃ | KHCO₃ | NaHCO₃ | NaHCO₃ | NaHCO₃ |
| Amount of base mmol | 2.5 | 5 | 10 | 14 | 5 | 7.5 | 10 |
| Addition of phase transfer catalyst | yes | yes | yes | yes | yes | yes | yes |
| Hydrogen pressure/MPa | 4 | 4 | 4 | 4 | 4 | 4 | 4 |
| Reaction temperature/°C | 90 | 90 | 90 | 90 | 90 | 90 | 90 |
| Reaction time/hr | 2.5 | 2.5 | 2.5 | 2.5 | 2.5 | 2.5 | 2.5 |
| Acid Base ratio 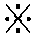 1 | 0.64 | 0.66 | 0.78 | 0.85 | 0.60 | 0.49 | 0.33 |
| TON 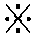 2 | 13,601 | 27,728 | 65,885 | 101,148 | 25,557 | 31,498 | 28,053 |
| Amount of formate formed mmol | 1.6 | 3.3 | 7.8 | 11.9 | 3 | 3.675 | 3.3 |

| | | | | | | | |
|---|---|---|---|---|---|---|---|
| 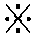 1: Amount (mol) of formic acid formed to amount (mol) of base charged 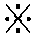 2: Turnover Number (amount (mol) of formate formed to amount (mol) of catalyst charged) | | | | | | | |

Examples 18 to 24, in which a formate was produced using the production method according to the first embodiment, show high TON (Turnover Number) and are excellent in the production efficiency for a formate. It could be confirmed that even when the base concentration was variously changed using potassium hydrogen carbonate and sodium hydrogen carbonate as bases, formic acid could be formed efficiently.

### [Examples 25 to 28]

1 mL of water was weighed out and put in a glass vial equipped with a stirring rod in a glovebox under an inert gas, 5 mmol of potassium hydrogen carbonate was added, and thereafter a solution obtained by mixing the Ru catalyst 1 in an amount in the range of 0.059 to 0.006 µmol and 54 µmol of methyltrioctylammonium chloride with 1 mL of toluene was added. Thereafter, the vial was placed in an autoclave, and the autoclave was sealed and taken out of the glovebox.

The autoclave was heated to 90°C while being stirred. When the temperature reached the target temperature, the autoclave was pressurized to 4 MPa with hydrogen. The reaction mixture was stirred for a time in the range of 18 to 48 hours, thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 µL of the aqueous solution of the lower layer was collected and dissolved in 500 µL of deuterated water, 300 µL of dimethyl sulfoxide was added as an internal standard, and then potassium formate was quantitively determined by ¹H NMR analysis.

Examples 25 to 28 are described in Table 5.

### [Table 5]

**Table 5**

| Test No. | Example 25 | Example 26 | Example 27 | Example 28 |
|---|---|---|---|---|
| Catalyst | Catalyst No. 1 | Catalyst No. 1 | Catalyst No. 1 | Catalyst No. 1 |
| Amount of catalyst µmol | 0.059 | 0.029 | 0.012 | 0.006 |
| Solvent | Water/toluene 1 mL/1 mL | Water/toluene 1 mL/1 mL | Water/toluene 1 mL/1 mL | Water/toluene 1 mL/1 mL |
| CO₂ source | KHCO₃ | KHCO₃ | KHCO₃ | KHCO₃ |
| Kind of base | KHCO₃ | KHCO₃ | KHCO₃ | KHCO₃ |
| Amount of base mmol | 5 | 5 | 5 | 5 |
| Addition of phase transfer catalyst | yes | yes | yes | yes |
| Hydrogen pressure/MPa | 4 | 4 | 4 | 4 |
| Reaction temperature/°C | 90 | 90 | 90 | 90 |
| Reaction time/hr | 18 | 18 | 18 | 48 |
| Acid Base ratio 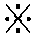 1 | 0.67 | 0.64 | 0.70 | 0.57 |
| TON 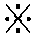 2 | 59,996 | 109,224 | 296,756 | 475,000 |
| Amount of formate formed mmol | 3.35 | 3.2 | 3.5 | 2.85 |

| | | | | |
|---|---|---|---|---|
| 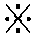 1: Amount (mol) of formic acid formed to amount (mol) of base charged 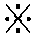 2: Turnover Number (amount (mol) of formate formed to amount (mol) of catalyst charged) | | | | |

Examples 25 to 28, in which a formate was produced using the production method according to the first embodiment, show high TON (Turnover Number) and are excellent in the production efficiency for a formate. It could be confirmed that even when the catalyst concentration was changed variously, a formate could be formed efficiently.

### [Examples 29 to 43]

1 mL of water was weighed out and put in a glass vial equipped with a stirring rod in a glovebox under an inert gas, 5 mmol of potassium hydrogen carbonate was added, and thereafter a solution obtained by mixing 0.12 µmol of the Ru catalyst 1 and 54 µmol of methyltrioctylammonium chloride with 1 mL of toluene was added. Thereafter, the vial was placed in an autoclave, and the autoclave was sealed and taken out of the glovebox.

The autoclave was heated to a temperature in the range of 65°C to 120°C while being stirred. When the temperature reached the target temperature, the autoclave was pressurized to a pressure in the range of 0.5 to 6 MPa with hydrogen. The reaction mixture was stirred for 16 hours, thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 µL of the aqueous solution of the lower layer was collected and dissolved in 500 µL of deuterated water, 300 µL of dimethyl sulfoxide was added as an internal standard, and then potassium formate was quantitively determined by ¹H NMR analysis.

Examples 29 to 43 are described in Table 6 and Table 7.

### [Table 6]

**Table 6**

| Test No. | Example 29 | Example 30 | Example 31 | Example 32 | Example 33 | Example 34 | Example 35 | Example 36 | Example 37 | Example 38 |
|---|---|---|---|---|---|---|---|---|---|---|
| Catalyst | Catalyst No. 1 | Catalyst No. 1 | Catalyst No. 1 | Catalyst No. 1 | Catalyst No. 1 | Catalyst No. 1 | Catalyst No. 1 | Catalyst No. 1 | Catalyst No. 1 | Catalyst No. 1 |
| Amount of catalyst µmol | 0.12 | 0.12 | 0.12 | 0.12 | 0.12 | 0.12 | 0.12 | 0.12 | 0.12 | 0.12 |
| Solvent | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL | Water/toluene 1 mL/1 mL |
| CO₂ source | KHCO₃ | KHCO₃ | KHCO₃ | KHCO₃ | KHCO₃ | KHCO₃ | KHCO₃ | KHCO₃ | KHCO₃ | KHCO₃ |
| Kind of base | KHCO₃ | KHCO₃ | KHCO₃ | KHCO₃ | KHCO₃ | KHCO₃ | KHCO₃ | KHCO₃ | KHCO₃ | KHCO₃ |
| Amount of base mmol | 5 | 5 | 5 | 5 | 5 | 5 | 5 | 5 | 5 | 5 |
| Addition of phase transfer catalyst | yes | yes | yes | yes | yes | yes | yes | yes | yes | yes |
| Hydrogen pressure/MPa | 0.5 | 1 | 2 | 4 | 6 | 0.5 | 1 | 2 | 4 | 5 |
| Reaction temperature/°C | 65 | 65 | 65 | 65 | 65 | 90 | 90 | 90 | 90 | 90 |
| Reaction time/hr | 16 | 16 | 16 | 16 | 16 | 16 | 16 | 16 | 16 | 16 |
| Acid Base ratio 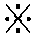 1 | 0.34 | 0.5 | 0.63 | 0.73 | 0.74 | 0.35 | 0.36 | 0.6 | 0.73 | 0.89 |
| TON 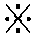 2 | 14306 | 21310 | 26598 | 31,089 | 31,360 | 14,777 | 14,961 | 25,287 | 30,955 | 37,997 |
| Amount of formate formed mmol | 1.7 | 2.5 | 3.15 | 3.65 | 3.7 | 1.75 | 1.8 | 3 | 3.65 | 4.45 |

| | | | | | | | | | | |
|---|---|---|---|---|---|---|---|---|---|---|
| 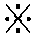 1: Amount (mol) of formic acid formed to amount (mol) of base charged 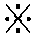 2: Turnover Number (amount (mol) of formate formed to amount (mol) of catalyst charged) | | | | | | | | | | |

### [Table 7]

**Table 7**

| Test No. | Example 39 | Example 40 | Example 41 | Example 42 | Example 43 |
|---|---|---|---|---|---|
| Catalyst | Catalyst No. 1 | Catalyst No. 1 | Catalyst No. 1 | Catalyst No. 1 | Catalyst No. 1 |
| Amount of catalyst µmol | 0.12 | 0.12 | 0.12 | 0.12 | 0.12 |
| Solvent | Water/toluene 1 mL/1 mL | Water/toluene 1 mL/1 mL | Water/toluene 1 mL/1 mL | Water/toluene 1 mL/1 mL | Water/toluene 1 mL/1 mL |
| CO₂ source | KHCO₃ | KHCO₃ | KHCO₃ | KHCO₃ | KHCO₃ |
| Kind of base | KHCO₃ | KHCO₃ | KHCO₃ | KHCO₃ | KHCO₃ |
| Amount of base mmol | 5 | 5 | 5 | 5 | 5 |
| Addition of phase transfer catalyst | yes | yes | yes | yes | yes |
| Hydrogen pressure/MPa | 0.5 | 1 | 2 | 4 | 5.3 |
| Reaction temperature/°C | 120 | 120 | 120 | 120 | 120 |
| Reaction time/hr | 16 | 16 | 16 | 16 | 16 |
| Acid Base ratio 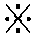 1 | 0.008 | 0.19 | 0.34 | 0.67 | 0.82 |
| TON 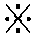 2 | 361 | 7,979 | 14,363 | 28,274 | 34,862 |
| Amount of formate formed mmol | 0.04 | 0.95 | 1.7 | 3.35 | 4.1 |

| | | | | | |
|---|---|---|---|---|---|
| 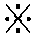 1: Amount (mol) of formic acid formed to amount (mol) of base charged 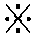 2: Turnover Number (amount (mol) of formate formed to amount (mol) of catalyst charged) | | | | | |

Examples 29 to 43, in which a formate was produced using the production method according to the first embodiment, show high TON (Turnover Number) and are excellent in the production efficiency for a formate. It could be confirmed that even when the reaction temperature and hydrogen pressure were changed variously, a formate could be formed efficiently.

### [Examples 44 to 48]

1 mL of water was weighed out and put in a glass vial equipped with a stirring rod in a glovebox under an inert gas, potassium hydrogen carbonate was added in an amount in the range of 5 mmol, and thereafter a solution obtained by mixing 0.13 µmol of the Ru catalyst 1 and benzyltriethylammonium chloride, tetrabutylammonium hydroxide, tetrabutylammonium fluoride, tetrabutylammonium bromide or tetrabutylammonium iodide as a phase transfer catalyst in an amount in the range of 42 to 57 µmmol with 1 mL of toluene was added. Thereafter, the vial was placed in an autoclave, and the autoclave was sealed and taken out of the glovebox.

The autoclave was heated to 90°C while being stirred. When the temperature reached the target temperature, the autoclave was pressurized to 4.5 MPa with hydrogen. The reaction mixture was stirred for 18 hours, thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 µL of the aqueous solution of the lower layer was collected and dissolved in 500 µL of deuterated water, 300 µL of dimethyl sulfoxide was added as an internal standard, and then potassium formate was quantitively determined by ¹H NMR analysis.

Examples 44 to 48 are described in Table 8.

### [Table 8]

**Table 8**

| Test No. | Example 44 | Example 45 | Example 46 | Example 47 | Example 48 |
|---|---|---|---|---|---|
| Catalyst | Catalyst No. 1 | Catalyst No. 1 | Catalyst No. 1 | Catalyst No. 1 | Catalyst No. 1 |
| Amount of catalyst µmol | 0.13 | 0.13 | 0.13 | 0.13 | 0.13 |
| Solvent | Water/toluene 1 mL/1 mL | Water/toluene 1 mL/1 mL | Water/toluene 1 mL/1 mL | Water/toluene 1 mL/1 mL | Water/toluene 1 mL/1 mL |
| CO₂ source | KHCO₃ | KHCO₃ | KHCO₃ | KHCO₃ | KHCO₃ |
| Kind of base | KHCO₃ | KHCO₃ | KHCO₃ | KHCO₃ | KHCO₃ |
| Amount of base mmol | 5 | 5 | 5 | 5 | 5 |
| Kind of phase transfer catalyst | Benzyltriethylammonium chloride | Tetrabutylammonium hydroxide | Tetrabutylammonium fluoride | Tetrabutylammonium bromide | Tetrabutylammonium iodide |
| Amount of phase transfer catalyst µmol | 50 | 57 | 50 | 42 | 49 |
| Hydrogen pressure/MPa | 4.5 | 4.5 | 4.5 | 4.5 | 4.5 |
| Reaction temperature/°C | 90 | 90 | 90 | 90 | 90 |
| Reaction time/hr | 18 | 18 | 18 | 18 | 18 |
| Acid Base ratio 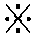 1 | 0.21 | 0.48 | 0.69 | 0.38 | 0.94 |
| TON 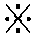 2 | 8,408 | 19,692 | 27,360 | 15,276 | 39,039 |
| Amount of formate formed mmol | 1.1 | 2.4 | 3.5 | 1.9 | 4.7 |

| | | | | | |
|---|---|---|---|---|---|
| 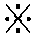 1: Amount (mol) of formic acid formed to amount (mol) of base charged 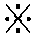 2: Turnover Number (amount (mol) of formate formed to amount (mol) of catalyst charged) | | | | | |

Examples 44 to 48, in which a formate was produced using the production method according to the first embodiment, show high TON (Turnover Number) and are excellent in the production efficiency for a formate. It could be confirmed that even when the kind of the phase transfer catalyst was changed, a formate can be formed efficiently.

### [Example 49]

1 mL of toluene was weighed out and put in a glass vial equipped with a stirring rod in a glovebox under an inert gas, 0.12 µmol of a ligand A and 0.12 µmol of [RuHCl(PPh₃)₃(CO)] were added thereto, and the toluene solution was heated to 65°C and stirred for 3 hours. Thereafter, 54 µmol of methyltrioctylammonium chloride was added to the toluene solution, 1 mL of water and 5 mmol of potassium hydrogen carbonate were added, the vial was placed in an autoclave, and the autoclave was sealed and taken out of the glovebox.

The autoclave was heated to 90°C while being stirred. When the temperature reached the target temperature, the autoclave was pressurized to 4 MPa with hydrogen. The reaction mixture was stirred for 18 hours, thereafter the reaction mixture was cooled with an ice bath, and pressure was carefully released. An upper layer of the solution after the reaction was removed, and an aqueous solution of a lower layer containing potassium formate and unreacted potassium hydrogen carbonate remained. 100 µL of the aqueous solution of the lower layer was collected and dissolved in 500 µL of deuterated water, 300 µL of dimethyl sulfoxide was added as an internal standard, and then potassium formate was quantitively determined by ¹H NMR analysis.

Example 49 is described in Table 9.

### [Table 9]

**Table 9**

| Test No. | Example 49 |
|---|---|
| Catalyst | Catalyst No. 1 |
| Amount of catalyst µmol | 0.12 |
| Solvent | Water/toluene 1 mL/1 mL |
| CO₂ source | KHCO₃ |
| Kind of base | KHCO₃ |
| Amount of base mmol | 5 |
| Addition of phase transfer catalyst | yes |
| Hydrogen pressure/MPa | 4 |
| Reaction temperature/°C | 90 |
| Reaction time/hr | 18 |
| Acid Base ratio 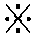 1 | 0.74 |
| TON 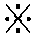 2 | 30,655 |
| Amount of formate formed mmol | 3.7 |
| Technique of preparing catalyst | Ligand and Ru precursor are dissolved in toluene solution to be used in reaction |

| | |
|---|---|
| 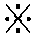 1: Amount (mol) of formic acid formed to amount (mol) of base charged 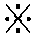 2: Turnover Number (amount (mol) of formate formed to amount (mol) of catalyst charged) | |

It could be confirmed that even when a series of reactions from the synthesis of an Ru catalyst to the synthesis of a formate were conducted in the same glass vial, the Example show high TON (Turnover Number) and is excellent in the production efficiency for a formate.

### INDUS TRIAL APPLICABILITY

According to the invention, a method for producing a formate, in which a formate as a precursor of formic acid is produced in high yield and a catalyst can be reused, a method for producing formic acid, a catalyst for the production of a formate, and a ruthenium complex that can be used as a catalyst converting hydrogen to a formate in high efficiency, can be provided.

The invention was described in detail and with reference to specific embodiments. However, it is obvious to those ordinarily skilled in the art that various modifications and alterations can be made without departing from the spirit and scope of the invention.

The invention is based on Japanese Patent Application (Japanese Patent Application No. 2020-148562) filed on September 3, 2020, Japanese Patent Application (Japanese Patent Application No. 2021-021223) filed on February 12, 2021, Japanese Patent Application (Japanese Patent Application No. 2021-021224) filed on February 12, 2021, Japanese Patent Application (Japanese Patent Application No. 2021-021225) filed on February 12, 2021, Japanese Patent Application (Japanese Patent Application No. 2021-079887) filed on May 10, 2021, and Japanese Patent Application (Japanese Patent Application No. 2021-083416) filed on May 17, 2021, the disclosures of which are incorporated herein by reference.

## Claims

1. A method for producing a formate, the method comprising reacting hydrogen with carbon dioxide, a hydrogen carbonate or a carbonate using a catalyst in the presence of a solvent,
wherein the reaction is a two-phase system in which an organic solvent and an aqueous solvent are present in a separated state in the solvent, and
the catalyst is at least one selected from a ruthenium complex represented by the following formula (1), a tautomer or stereoisomer thereof, or a salt compound of the complex, tautomer or stereoisomer: 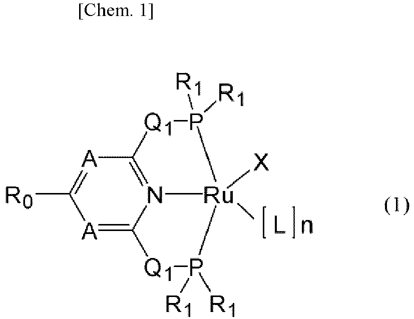 wherein R₀ represents a hydrogen atom or an alkyl group,
Q₁ each independently represents CH₂, NH or O,
R₁ each independently represents an alkyl group or an aryl group (provided that when Q₁ represents NH or O, at least one of R₁ represents an aryl group),
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.

2. The method for producing a formate according to claim 1, wherein the ruthenium complex represented by the formula (1) is a ruthenium complex represented by the following formula (3): 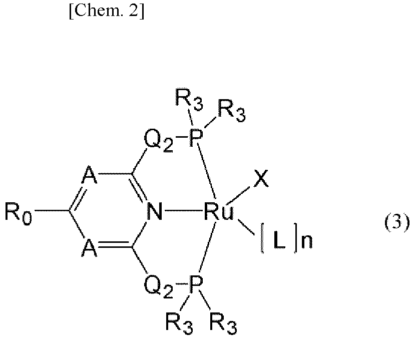 wherein R₀ represents a hydrogen atom or an alkyl group,
Q₂ each independently represents NH or O,
R₃ each independently represents an aryl group,
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.

3. The method for producing a formate according to claim 1, wherein the R₁ represents a phenyl group.

4. The method for producing a formate according to claim 2, wherein the R₃ represents a phenyl group.

5. The method for producing a formate according to claim 4, wherein the A represents CH, and the Q₂ represents NH.

6. The method for producing a formate according to any one of claims 1 to 5, wherein the R₀ represents a hydrogen atom or a methyl group.

7. The method for producing a formate according to any one of claims 1 to 6, wherein the X represents a chlorine atom.

8. The method for producing a formate according to any one of claims 1 to 7, wherein the n represents 1 to 3, and the L each independently represents a hydrogen atom, carbon monoxide or triphenylphosphine.

9. The method for producing a formate according to any one of claims 1 to 8, wherein the organic solvent contains toluene or dioxane.

10. The method for producing a formate according to any one of claims 1 to 9, wherein an ammonium salt is further used as a phase transfer catalyst.

11. The method for producing a formate according to any one of claims 1 to 10, wherein a ligand represented by the following formula (4) is further added: 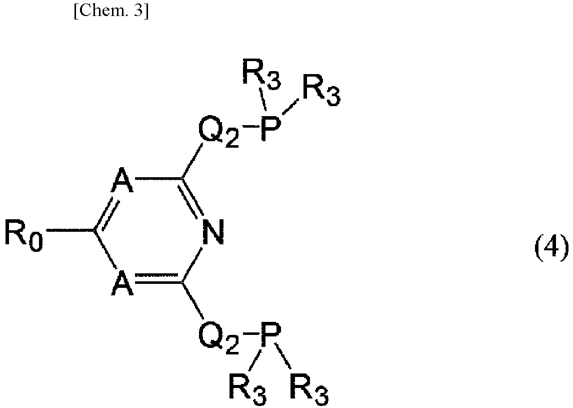 wherein R₀ represents a hydrogen atom or an alkyl group,
Q₂ each independently represents NH or O,
R₃ each independently represents an aryl group,
A each independently represents CH, CRs or N, and R₅ represents an alkyl group, an aryl group, an aralkyl group, an amino group, a hydroxy group or an alkoxy group.

12. A method for producing formic acid, the method comprising:
a step of producing a formate by the method for producing a formate according to any one of claims 1 to 11; and
a second step of protonating at least a part of the formate to form formic acid.

13. A catalyst for the production of a formate, for use in the production of a formate by a reaction of hydrogen with carbon dioxide, a hydrogen carbonate or a carbonate, the catalyst comprising a ruthenium complex represented by the following formula (2): 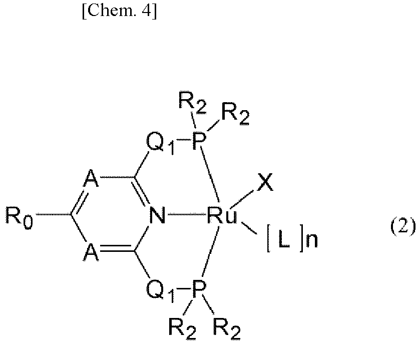 wherein R₀ represents a hydrogen atom or an alkyl group,
Q₁ each independently represents CH₂, NH or O,
R₂ each independently represents an alkyl group or an aryl group (provided that at least one of R₂ represent an aryl group),
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.

14. A ruthenium complex represented by the following formula (3): 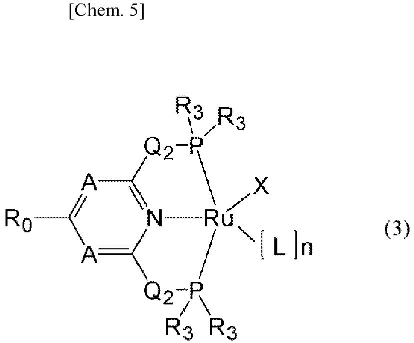 wherein R₀ represents a hydrogen atom or an alkyl group,
Q₂ each independently represents NH or O,
R₃ each independently represents an aryl group,
A each independently represents CH, CRs or N, R₅ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.

15. The ruthenium complex according to claim 14, wherein the R₃ represents a phenyl group.

16. The ruthenium complex according to claim 14 or 15, wherein the A represents CH and the Q₂ represents NH.

17. The ruthenium complex according to any one of claims 14 to 16, wherein the R₀ represents a hydrogen atom or a methyl group.

18. The ruthenium complex according to any one of claims 14 to 17, wherein the X represents a chlorine atom.

19. The ruthenium complex according to any one of claims 14 to 18, wherein the n represents 1 to 3, and the L each independently represents a hydrogen atom, carbon monoxide or triphenylphosphine.
